(19) Europäisches Patentamt / European Patent Office / Office européen des brevets

(11) **EP 3 935 100 B1**

(12) **EUROPEAN PATENT SPECIFICATION**

(45) Date of publication and mention of the grant of the patent:
**12.07.2023 Bulletin 2023/28**

(21) Application number: **19709927.8**

(22) Date of filing: **08.03.2019**

(51) International Patent Classification (IPC):
***C08G 64/30*** *(2006.01)* ***C08G 73/02*** *(2006.01)*

(52) Cooperative Patent Classification (CPC):
**C08G 64/30; C08G 64/0241; C08G 64/025; C08G 73/0233;** C08G 14/06

(86) International application number:
**PCT/EP2019/055862**

(87) International publication number:
**WO 2020/182277 (17.09.2020 Gazette 2020/38)**

# (54) NOVEL EXPANDING COPOLYMERS

NEUARTIGE EXPANDIERBARE COPOLYMERE

NOUVEAUX COPOLYMÈRES EXPANSIBLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.01.2022 Bulletin 2022/02**

(73) Proprietor: **Polymer Competence Center Leoben Gmbh**
**8700 Leoben (AT)**

(72) Inventors:
- **WIESBROCK, Frank**
  **8770 St. Michael in Obersteiermark (AT)**
- **WINDBERGER, Matthias Sebastian**
  **7212 Forchtenstein (AT)**

(74) Representative: **Patentanwaltskanzlei Matschnig & Forsthuber OG**
**Biberstraße 22**
**Postfach 36**
**1010 Wien (AT)**

(56) References cited:
**EP-A1- 2 560 227**
**WO-A1-2013/148408**
**WO-A1-2018/229415**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 935 100 B1

## Description

The present invention relates to a novel expandable, polymerizable composition, as described by the present claims a polymerization product of said expandable, polymerizable composition as well as a method for manufacturing the polymerization product. The invention also relates to a sealant, adhesive, coating, binding agent or dental filling comprising said expandable, polymerizable composition as well as the use of said expandable, polymerizable composition as/in sealants, adhesives, coatings, binding agents or dental fillings.

Volume shrinkage that occurs during the process of polymerization is often neglected and highly under-represented in the literature. The shrinkage during polymerization originates from the change in the bonding distance from van-der-Waals distances in the monomer (3.4 Å) to covalent distances in the polymer (1.5 Å). Values for polymerization shrinkage can be as high as 50% for polycondensation polymerizations or as low as 2-5% in ring-opening polymerizations.

A great number of currently used polymerizable compositions are predominantly based on epoxides. These epoxy-based compositions exhibit volumetric shrinkage during polymerization resulting in delamination, cracking and formation of voids in the resin. In addition to the shrinking, epoxy-based polymers also have other known disadvantages including a high moisture uptake and limited stability at higher temperatures.

In order to find a solution to this problem, different strategies to reduce the volume shrinkage during polymerizations have been developed. These strategies are mainly based on three approaches: (A) change of polymerization conditions (photopolymerization or thermally cured polymers), (B): addition of fillers or additives, and (C): modification of the structure of the monomer or oligomer.

For example, spiroorthocompounds (SOC compounds) were reported as the monomers with exceptionally high volumetric expansion upon polymerization (T. Takata, T. Endo: Recent advances in the development of expanding monomers: Synthesis, polymerization and volume change. Prog. Polym. Sci. 1993, 18(5), 839-870). However, SOC compounds have the disadvantage of being water sensitive. Another potential issue considering industrial applications is the fact that the synthesis of numerous spiroortho monomers in the literature involves various compounds that are prohibited by REACH (namely the registration, evaluation, authorization and restriction of chemicals).

Another set of compounds that was reported to lead to volume expansion upon curing are benzoxazine-based phenolic resins (H. Ishida, H. Y. Low: A Study on the Volumetric Expansion of Benzoxazine-Based Phenolic Resin. Macromolecules 1997, 30, 1099-1106). Various benzoxazine monomers can be prepared from bisphenolic units (most frequently Bisphenol A), formaldehyde, and primary amines. However, a huge difference among different amine-based benzoxazines in their volumetric expansion characteristics was observed. Even if poly(benzoxazine)s can overcome many of the disadvantages reported for epoxy-based polymers, the methods for producing poly(benzoxazine)s require high temperatures and the resulting polymers are typically brittle, thus, limiting their spectrum of applications.

The volume expansion during the polymerization of cyclic carbonates containing norbornene units (NBC) with an amine-based initiator was reported by Murayama (M. Murayama: Anionic Ring-Opening Polymerization of a Cyclic Carbonate Having a Norbornene Structure with Amine Initiators. Macromolecules 1998, 31, 919-923). The polymerization of pure NBC has been reported to lead to an expansion of 8.2%.

Cho and coworkers studied the effect on the shrinkage of incorporating various lactams into epoxy resins during copolymerization (L.W. Chen, C.H. Twu, C.S. Cho: Physical properties and shrinkage of epoxy resins cured with lactams. J. Polym. Res. 1997, 4, 65-72). The shrinkage decreased with increasing lactam content and with increasing lactam ring size.

Furthermore, it was reported that polyurethane acrylates with different amounts of stearyl alcohols effect the volume shrinkage (L. Qin, J. Nie, Y. He: Synthesis and properties of polyurethane acrylate modified by different contents of stearyl alcohol. J. Coat. Technol. Res. 2015, 12, 197-204). Specifically, polyurethane acrylates modified by a higher stearyl alcohol content exhibit lower volume shrinkage.

As the polymerization shrinkage is directly related to the conversion of reactive double bonds, novel photopolymerizable formulations based on dimethylacrylate monomers with bulky substituent groups were shown to exhibit lower shrinkage upon polymerization (J. Ge, M. Trujillo, J. Stansbury: Synthesis and photopolymerization of low shrinkage methacrylate monomers containing bulky substituent groups. Dent. Mater. 2005, 21, 1163-1169).

Nie and coworkers studied the effect of temperature on the volume shrinkage during the photopolymerization of tri(ethylene glycol) dimethacrylate (B. Lu, P. Xiao, M. Sun, J. Nie: Reducing volume shrinkage by low-temperature photopolymerization. J. Appl. Polym. Sci. 2007, 104, 1126-1130). They found that the optimum temperature for the photocuring is -40 °C as the total conversion after the dark reaction is similar to the one observed at room temperature, while beneficially the shrinkage was significantly lower.

Another study demonstrated that the shrinkage during photopolymerization of (meth)acrylate coatings depends on double bond conversion and the concentration of double bonds: the monomer was mainly responsible for shrinkage due to the inherent high concentration of double bonds (Y. Jian, Y. He, T. Jiang, C. Li, W. Yang, J. Nie: Volume shrinkage of UV-curable coating formulation investigated by real-time laser reflection method. J. Coat. Technol. Res. 2013, 10, 231-237).

Another approach relates to the use of prepolymers to prevent volume shrinkage. The synthesis of novel types of hyperbranched polyesters with a flexible ethoxylated Bisphenol A structure and terminal hydroxyl groups was reported by Serra and coworkers (T. Li, H. Qin, Y. Liu, Y. Zhong, Y. Yu, A. Serra: Hyperbranched polyester as additives in filled and unfilled epoxy-novolac systems. Polymer 2012, 53(25), 5864-5872).

The effect of the $TiO_2$ filler on the volume shrinkage of epoxy resin composites was studied by Thomas and coworkers (J. Parameswaranpillai, A. George, J. Pionteck, S. Thomas: Investigation of Cure Reaction, Rheology, Volume Shrinkage and Thermomechanical Properties of Nano-TiO2 Filled Epoxy/DDS Composites. J. Polym. 2013, 183463:1-183463:17).

EP 2 560 227 A1 discloses the synthesis of a polymeric electrolyte membrane from a polymerizable mixture comprising a benzoxazine compound. WO 2018/229415 A1 discloses a sulphurized benzoxazine compound for use in the synthesis of polybenzoxazine.

There is a need for alternative or improved expandable, polymerizable compositions that result in polymers having an improved and tunable expansion behavior during polymerization.

It is an object of the present invention to provide a novel expandable, polymerizable composition based on benzoxazines showing improved expansion properties, in particular, showing high, adjustable and reproducible expansion rates during polymerization. It is another object of the present invention to provide a novel expandable, polymerizable composition based on benzoxazines, which results in polymers having a wide spectrum of applications.

These objects are achieved by a novel expandable, polymerizable composition comprising at least one benzoxazine and at least one cyclic carbonate,characterised in that the benzoxazine units comprised in the composition are benzoxazine units which are crosslinkable with one another.

The present invention is based on the surprising finding that copolymerizing benzoxazine monomers with cyclic carbonate monomers results in novel copolymers having unforeseeably high expansion rates, wherein the properties (e.g. solid/brittle, solid/soft, rubbery) of the resulting copolymers can be easily and reproducibly tuned/adjusted, depending on the ratio of the benzoxazine equivalents/cyclic carbonate equivalents present in the composition and copolymer, respectively. Thus, the novel copolymers provide significant benefits over the expandable polymers known in the art. Furthermore, the novel expandable, polymerizable compositions and poly(benzoxazine)-co-poly(cyclic carbonate)-polymers obtained therewith have a great potential for offering a wide spectrum of applications. As will be described in more detail below, the copolymer can be prepared by the copolymerization of benzoxazines and cyclic carbonates at a temperature sufficient to initiate copolymerization, wherein both types of monomers undergo ring-opening polymerization.

These surprising findings are evidenced by the experimental data given in the examples below. The results presented in the experimental data show that for most of the copolymers that were tested expansion rates of more than 25% or of even more than 30% were observed. To the inventors' best knowledge, such high expansion rates have never been reported so far for expandable polymers, thus, exceeding the expansion rates of known expandable polymers by more than 300%.

One aspect of the present invention is the use of an expandable, polymerizable composition comprising at least one benzoxazine and at least one cyclic carbonate in the manufacture of a poly(benzoxazme)-co-poly(cycliccarbonate), wherein the expandable, polymerizable composition shows volumetric expansion during copolymerization upon a heat stimulus. In a further aspect, the one benzoxazine used is a compound of general Formula (I) :

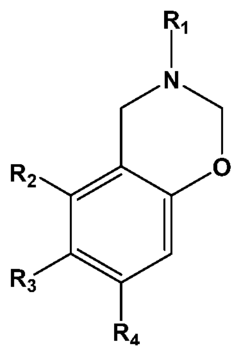

**Formula (I)**

wherein $R_1$, $R_2$, $R_3$, and $R_4$ are each independently H, $CH_3$, $C_2$-$C_{15}$ straight, branched or cyclic alkyl optionally substituted with halogens, alkenyl or alkynyl groups, alkaryl groups, heteroalkyl groups, heteroaryl groups, hydroxy groups, disulfides, sulfonates, ether groups, thiolether groups, ester groups, carboxylic acid groups, amine groups, amide groups, azides, or benzoxazines, and $R_2$ can be linked with $R_3$ to form a cyclic substituent on the benzene ring or $R_3$ can be linked with $R_4$ to form a cyclic substituent on the benzene ring.

The term "alkyl" as used herein refers to straight or branched hydrocarbon chains having a specified number of carbon atoms. For example, "$C_2$-$C_{15}$ straight, branched or cyclic alkyl" as recited in the claims refers to a straight, branched or cyclic alkyl group having at least 2 and at most 15 carbon atoms. Alkyl groups having a straight chain preferably represent $C_2$-$C_9$ alkyl. Useful branched alkyl chains, which preferably represent $C_3$-$C_7$ alkyl, are for example substituents of the composition isopropyl, isobutyl, neopentyl, and (1'-methyl)-hexyl. Useful cyclic alkyl chains, which preferably represent $C_4$-$C_{12}$ alkyl, are for example substituents of the composition cyclobutyl, cyclohexyl, and adamantyl .

As mentioned above, the $C_2$-$C_{15}$ straight, branched or cyclic alkyl is optionally substituted by one or more substituents independently selected from the group consisting of halogens, alkenyl or alkynyl groups, alkaryl groups, heteroalkyl groups, heteroaryl groups, hydroxy groups, disulfides, sulfonates, ether groups, thiolether groups, ester groups, carboxylic acid groups, amine groups, amide groups, azides, or benzoxazines.

The term "halogen" as used herein means the halogens chlorine, bromine, fluorine or iodine, wherein chlorine and bromine are preferred, as well as the *pseudo* halogens CN, $N_3$, OCN, NCO, CNO, SCN, NCS, SeCN , wherein $N_3$, NCO, and SCN, are preferred.

The term "alkenyl" as used herein refers to alkyl chains containing the specified number of carbon atoms and containing at least one carbon-carbon double bond, wherein alkyl is defined as above and may be a straight or a branched hydrocarbon chain or may bear ring closures. For example, $C_2$-$C_{25}$ alkenyl means an alkenyl containing at least 2, and at most 25, carbon atoms and containing at least one double bond. Straight alkenyl chains are preferred. Examples of "alkenyl" as used herein include but are not limited to 2-propenyl, Z- or *E*-propenyl, butadienyl, isoprenyl and cyclopropenylethyl as well as Z- *or E*-decenyl and *E*-octadec-9-enyl .

The term "alkinyl" as used herein refers to alkyl chains containing the specified number of carbon atoms and containing at least one carbon-carbon triple bond, wherein alkyl is defined as above and may be a straight or a branched hydrocarbon chain or may bear ring closures. For example, $C_2$-$C_{12}$ alkinyl means an alkinyl containing at least 2, and at most 12, carbon atoms and containing at least one triple bond. Representative examples of alkinyl include, but are not limited, to 1-propinyl, 2-propinyl, 3-butinyl and 2-pentinyl as well as 1-dodecinyl.

The term "alkaryl" as used herein refers to a group of the formula alkylene-aryl or arylene-alkyl. In particular, alkaryl relates to an aryl group attached to the benzoxazine ring via an alkylene group. The term "aryl" as used herein refers to an aromatic carbocyclic ring having a specified number of carbon atoms. For example, $C_5$-$C_6$ aryl refers to an aromatic ring having at least 5 and at most 6 carbon atoms. The term "aryl" also refers to a multicyclic group having more than one aromatic ring, such as $C_{10}$-$C_{14}$ aryl. Representative examples of aryl include, but are not limited to cyclopentadienyl, phenyl, naphthyl, anthracyl, phenanthryl. Exemplary alkaryl groups are from 7 to 16 carbon atoms, and include, but are not limited to benzyl, phenethyl, and isopropylbenzyl.

The term "heteroalkyl" as used herein refers to an "alkyl" group in which at least one carbon atom has been replaced with a heteroatom (e.g., an O, N, or S atom). The heteroalkyl may be, for example, primary, secondary, and tertiary amines, linear, branched, and cyclic thioethers, primary thiols, linear, branched, and cyclic ethers as well as carboxylic acids and esters derived thereof. In certain embodiments, the "heteroalkyl" may be 2-8 membered heteroalkyl, indicating that the heteroalkyl contains from 2 to 8 atoms selected from the group consisting of carbon, oxygen, nitrogen, and sulfur. In yet other embodiments, the heteroalkyl may be a 2-6 membered, 4-8 membered, or a 5-8 membered heteroalkyl group (which may contain for example 1 or 2 heteroatoms selected from the group oxygen and nitrogen).

The term "heteroaryl" as used herein refers to aromatic groups having one or more heteroatoms (O, S or N). Preferred heteroaryl residues have 5 or 6 ring members and include those derived from furan, imidazole, triazole, isothiazole, isoxazole, oxadiazole, oxazole, pyrazine, pyrazole, pyridazine, pyridine, pyrimidine, pyrroline, thiazole, thiophene, triazole, and tetrazole. A multicyclic group having one or more heteroatoms, wherein at least one ring of the group is heteroaromatic, is referred to as a "heteroaryl" as well; for example heteroaryl groups having 7 to 10 ring members and including those derived from indole, quinoline, isoquinoline and tetrahydroquinoline.

The terms "hydroxy", "disulfides", "sulfonates", "thioether" "ether", "ester" and "carboxylic acid" are art recognized. Exemplary disulfides include, but are not limited to methyl disulfanyl, ethyl disulfanyl, phenyl disulfanyl and benzyl disulfanyl. Exemplary sulfonates include, but are not limited to mesylate, tosylate, sodium methanesulfonate, potassium methanesulfonate, and sodium 2-methyl benzenesulfonate. Exemplary ether groups include, but are not limited to methoxy, ethoxy, benzoxy, and phenoxy.Exemplary thioether groups include, but are not limited to methylthiyl, ethylthiyl, and phenylthiyl. Exemplary ester groups include, but are not limited to formiates, acetates, propanoates, phthalates, and benzoates. Exemplary carboxylic acid groups include, but are not limited to formic acids, acetic acids, benzoic acids, phthalic acids, and fatty acids such as decanoic acids and dec-10'-enoic acid.

The terms "amine" and "amino" are art-recognized and refer to primary, secondary and tertiary amines, including, but not limited to ethylamine, benzylamine, ethylenediamine, isophoron diamine, diethylene triamine, tert-butyl amine, and triethylamine.

The term "amide" as used herein, represents a molecule having at least one identifiable amide group. Exemplary amides include, but are not limited to acetamidomethyl, propionamidomethyl, and benzamidomethyl.

The term "azide" as used herein refers to any group having the $N_3$ moiety therein. Exemplary azides include,

but are not limited to (4H-1,2,3-triazol-4-yl)methyl, (5-methyl-4H-1,2,3-triazol-4-yl)methyl, (5-ethyl-4H-1,2,3-triazol-4-yl)methyl, and (5-phenyl-4H-1,2,3-triazol-4-yl)methyl, as well as azidomethyl, azidoethyl, azidopropyl, 4-azidophenyl, and 4-azidobenzyl.

As used herein, the term "benzoxazine" relates to compounds and polymers comprising the characteristic benzoxazine ring. Exemplary benzoxazine substituents include, but are not limited to (2H-benzo[e][1,3]oxazin-3(4H)-yl)methyl, (5,6,7-trimethyl-2H-benzo[e][1,3]oxazin-3(4H)-yl)methyl, (7-amino-5-azido-6-hydroxy-2H-benzo[e][1,3]oxazin-3(4H)-yl)methyl, and 2-((2H-benzo[e][1,3]oxazin-3(4H)-yl)methyl)benzyl. For example, the synthesis of benzoxazine monomers of Formula (I) wherein one of $R_1$, $R_2$, $R_3$, and $R_4$ is $C_2$-$C_{15}$ straight, branched or cyclic alkyl substituted with a benzoxazine, may be based on a symmetric bisphenol compound or a dihydroxybenzene compound.

The term "substituted" refers to a chemical entity being substituted with one or more substituents. The term "optionally substituted" relating to a chemical entity means that the chemical entity may be substituted or not, i.e. both situations are included. The term "one or more substituents" refers to from one to the maximum number of substituents possible, depending on the number of available substitution sites.

In certain embodiments, $R_2$ can be linked with $R_3$ to form a cyclic substituent on the benzene ring. In other embodiments, $R_3$ can be linked with $R_4$ to form a cyclic substituent on the benzene ring. Exemplary cyclic substituents include, but are not limited to 3-allyl-1,3-oxazinane-5,6-diyl, 3-allyl-4-methyl-1,3-oxazinane-5,6-diyl, cyclohexane-1,2-diyl, and benzene-1,2-diyl. For example, by linking $R_2$ with $R_3$ or by linking $R_3$ with $R_4$ a cyclic substituent in form of a 4 -, 5- or 6- membered alkyl-ring may be formed.

In another aspect, the benzoxazine units comprised in the composition are benzoxazine units which are crosslinkable with each other. Preferably the benzoxazines are crosslinkable by thiol-ene crosslinking. In certain embodiments the crosslinkable benzoxazine is a compound of general Formula (II):

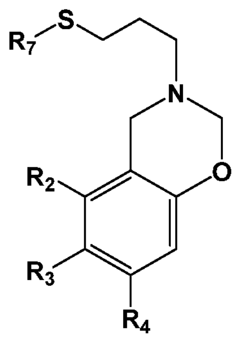

**Formula (II)**

wherein $R_2$, $R_3$, and $R_4$ are each independently H, $CH_3$, $C_2$-$C_{15}$ straight, branched or cyclic alkyl optionally substituted with halogens, alkenyl or alkynyl groups, alkaryl groups, heteroalkyl groups, heteroaryl groups, hydroxy groups, disulfides, sulfonates, ether groups, thioether groups, ester groups, carboxylic acid groups, amine groups, amide groups, azides, or benzoxazines, and $R_2$ can be linked with $R_3$ to form a cyclic substituent on the benzene ring or $R_3$ can be linked with $R_4$ to form a cyclic substituent on the benzene ring, with the proviso that $R_2$, $R_3$, and/or $R_4$ comprises at least one unsaturated bond, e.g. an olefinic bond, and

$R_7$ is $C_2$-$C_{15}$ straight, branched or cyclic alkyl optionally substituted with halogens, alkaryl groups, heteroalkyl groups, heteroaryl groups, hydroxy groups, disulfides, sulfonates, ether groups, thioether groups, ester groups, carboxylic acid groups, amine groups, amide groups and azides, with the proviso that $R_7$ comprises at least one thiol group in order to enable (UV-mediated) thiol-ene crosslinking.

UV-mediated crosslinking of monomers comprising available alkene and thiol functional groups via thiol-ene reactions (also known as "thiol-ene click chemistry") is well known to those skilled in the art and frequently used in the synthesis of crosslinked three-dimensional networks. In the present invention, the benzoxazine units may be crosslinked by a UV-initiated thiol-ene click reaction prior to the copolymerization. The copolymerization of the crosslinked benzoxazine units and the cyclic carbonate units, where expansion of the resulting copolymer takes place, is performed by applying heat at a temperature sufficient to initiate copolymerization, which temperature is e.g. a temperature of up to 140 °C in case of the present invention.

The at least one cyclic carbonate may be a compound of general Formula (**III**):

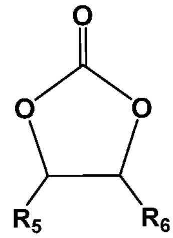

**Formula (III)**

wherein $R_5$ and $R_6$ are each independently H, $CH_3$, $C_2$-$C_{15}$ straight, branched or cyclic alkyl optionally substituted with halogens, alkenyl, heteroalkyl groups and hydroxy groups. In preferred embodiments, the cyclic carbonate is ethylene carbonate or propylene carbonate, preferably ethylene carbonate.

In specific embodiments, the benzoxazine may be derived from a dihydroxybenzene or bisphenol. Preferably the dihydroxybenzene is hydroquinone, and the bisphenol is selected from the group consisting of Bisphenol A, Bisphenol F, Bisphenol S, Bisphenol M, Bisphenol Z, and Bisphenol AP. Examples of such benzoxazines are given in the experimental part in the examples below.

The ratio of benzoxazine equivalents to cyclic carbonate equivalents in the composition is preferably from 99:1 to 1:99; more preferably, the ratio of benzoxazine equivalents to cyclic carbonate equivalents in the composition is from 99:1 to 30:70. Depending on the ratio, the characteristics (e.g. solid/brittle, solid/soft, rubbery) of the polymerization product obtained after polymerizing the composition according to the invention can be adjusted/tuned.

In other embodiments, the composition additionally comprises at least one reactive diluent. In specific embodiments, the reactive diluent is present at between 20% to 60% by weight of the benzoxazine. In specific embodiments, the reactive diluent is selected from the group consisting of 3-allyl-3,4-dihydro-2H-benzo[e][1,3]oxazine, 3-allyl-5-methyl-3,4-dihydro-2H-benzo[e][1,3]oxazine, 3-allyl-6-octyl-3,4-dihydro-2H-benzo[e][1,3]oxazine, and 3-allyl-6-nonyl-3,4-dihydro-2H-benzo [e] [1,3] oxazine.

The present invention also relates to a polymerization product of an expandable, polymerizable composition according to the present invention and described herein.

In certain embodiments, the polymerization product according to the invention comprises a poly(benzoxazine)-co-poly(cyclic carbonate) of the general formula **(IV):**

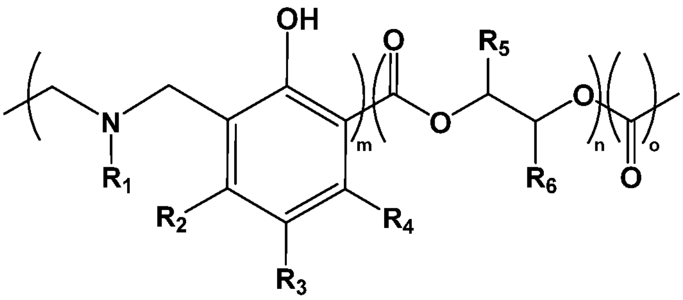

**Formula (IV)**

wherein

- $R_1$, $R_2$, $R_3$, and $R_4$ are each independently H, $CH_3$, $C_2$-$C_{15}$ straight, branched or cyclic alkyl optionally substituted with halogens, alkenyl or alkynyl groups, alkaryl groups, heteroalkyl groups, heteroaryl groups, hydroxy groups, disulfides, sulfonates, ether groups, thioether groups, ester groups, carboxylic acid groups, amine groups, amide groups, azides, or benzoxazine, and $R_2$ can be linked with $R_3$ to form a cyclic substituent on the benzene ring or $R_3$ can be linked with $R_4$ to form a cyclic substituent on the benzene ring;
- $R_5$ and $R_6$ are each independently H, $CH_3$, $C_2$-$C_{15}$ straight, branched or cyclic alkyl optionally substituted with halogens, alkenyl, heteroalkyl groups and hydroxy groups;
- m is an integer between 10 and 10'000;
- n is an integer between 10 and 6'700;

o is an integer between 0 and 1'000.

During copolymerization, both types of monomers, i.e. the benzoxazine monomers and cyclic carbonate monomers, undergo ring-opening polymerization as depicted below:

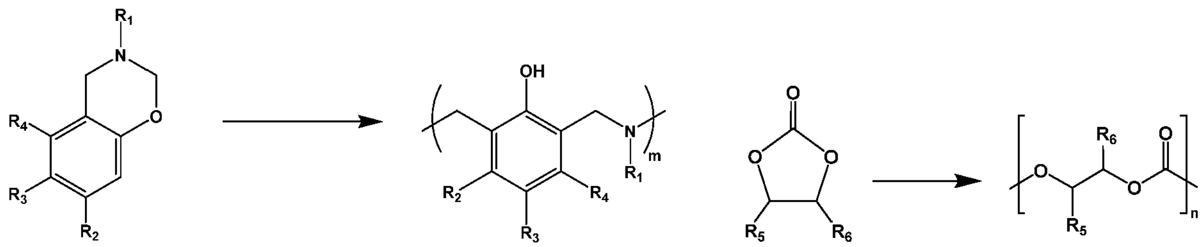

The copolymerization step for obtaining a poly(benzoxazine)-co-poly(cyclic carbonate) of the above-mentioned general formula (IV) is depicted below:

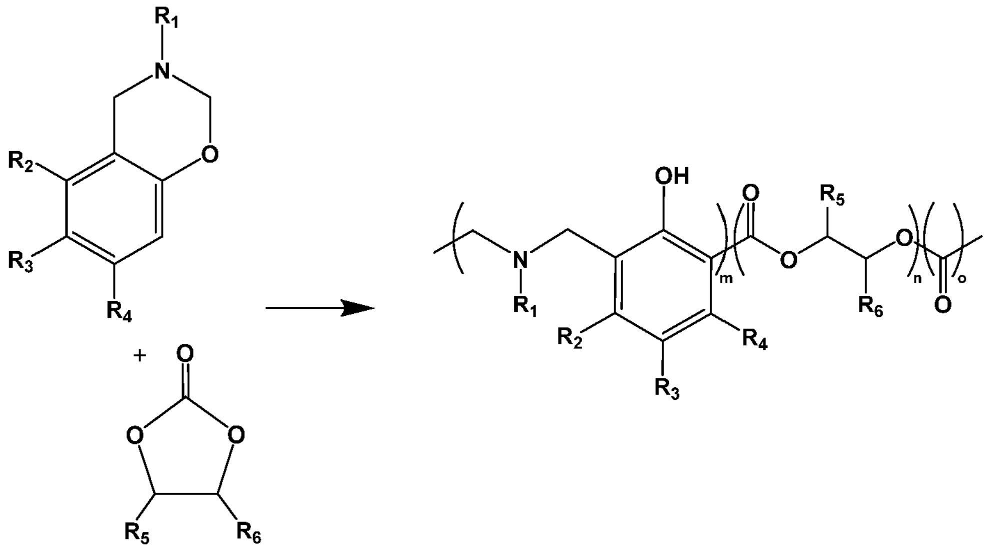

Formula (IV)

In certain embodiments, the polymerization product of the present invention comprises a poly(benzoxazine)-co-poly(cyclic carbonate) of the general formula (V):

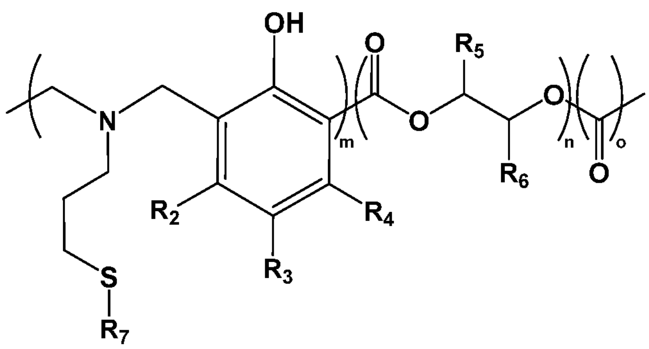

Formula (V)

wherein

$R_2$, $R_3$, and $R_4$ are each independently H, $CH_3$, $C_2$-$C_{15}$ straight, branched or cyclic alkyl optionally substituted with halogens, alkenyl or alkynyl groups, alkaryl groups, heteroalkyl groups, heteroaryl groups, hydroxy groups, disulfides, sulfonates, ether groups, thioether groups, ester groups, carboxylic acid groups, amine groups, amide groups, azides, or benzoxazine, and $R_2$ can be linked with $R_3$ to form a cyclic substituent on the benzene ring or $R_3$ can be linked with $R_4$ to form a cyclic substituent on the benzene ring, with the proviso that $R_2$, $R_3$, and/or $R_4$ comprises at least one unsaturated bond;

$R_7$ is $C_2$-$C_{15}$ straight, branched or cyclic alkyl optionally substituted with halogens, alkaryl groups, heteroalkyl groups, heteroaryl groups, hydroxy groups, disulfides, sulfonates, ether groups, thioether groups, ester groups, carboxylic acid groups, amine groups, amide groups and azides, with the proviso that $R_7$ comprises at least one thiol group, in order to enable (UV-mediated) thiol-ene crosslinking.

$R_5$ and $R_6$ are each independently H, $CH_3$, $C_2$-$C_{15}$ straight, branched or cyclic alkyl optionally substituted with halogens, alkenyl, heteroalkyl groups and hydroxy groups;

m is an integer between 10 and 10'000;

n is an integer between 10 and 6'700;

o is an integer between 0 and 1000.

The two-step polymerization for obtaining a poly(benzoxazine)-co-poly(cyclic carbonate) of the above-mentioned general formula (V) is depicted below:

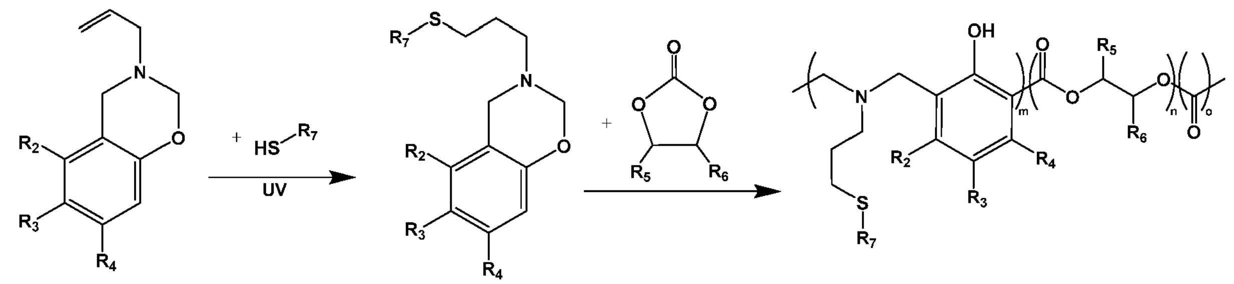


Formula (V)

In these embodiments, the benzoxazine units are first be crosslinked by a UV-initiated thiol-ene click reaction prior to copolymerizing the crosslinked benzoxazine units and the cyclic carbonates. The copolymerization, during which expansion of the resulting copolymer takes place, is performed by applying heat at a temperature sufficient to initiate copolymerization, which temperature is e.g. a temperature of up to 140 °C in case of the present invention.

In a further aspect, the present invention also relates to a sealant, an adhesive, a coating, a binding agent or a dental filling comprising an expandable polymerizable composition as described herein. In yet another aspect, the present invention also relates to the use of an expandable, polymerizable composition as described herein as/in sealants, adhesives, coatings, binding agents or dental fillings. For example, the expandable polymerizable composition may be useful in the building industry, e.g. as casting compositions and sealing agents; in the casting industry, e.g. as binders and moulding mixtures; in the coating industry, e.g. for coating of objects; in the wood processing industry, e.g. for the preparation of pressed wood products; in the automotive industry; in the electronic industry, e.g. as insulating materials; in the adhesives industry; and in the field of medical/dental products, e.g. dental fillings.

In a further aspect, the present invention relates to a precured, expandable, polymerizable composition obtained by crosslinking crosslinkable benzoxazines comprised in an expandable polymerizable composition as described herein. In a further aspect, the present invention also relates to the use of this precured, expandable, polymerizable composition, as an expandable filling element for filling spaces in devices, wherein the precured, expandable composition has a pre-defined form, in particular, but not limited to, bars, sticks, cylinders or building blocks, wherein the precured, expandable composition shows volumetric expansion during copolymerization upon a heat stimulus. In specific embodiments, the volumentric expansion of the pre-formed and precured composition may result in a geometric alignment with the space to be filled. Said pre-defined forms of the precured, expandable, polymerizable composition may have dimensions in the milli-, centi-, or meter range. Specifically, said precured, expandable, polymerizable composition may be used as a support and fixation element for windings of electrical machines or may serve as a winding insulation barrier for electrical equipment. That is, due to the volumetric expansion, precise geometric alignment of the resulting expanded copolymer

to the surrounding material occurs and compensates the shrinkage and assembling tolerances of those materials, which renders excellent stability and, additionally, insulating properties and form fit support of the windings or laminated electromagnetic materials (see also chapter 4 of the applications examples below).

In yet another aspect, the present invention also relates to a process for manufacturing a polymerization product, comprising the step of heating an expandable, polymerizable composition as described herein to a temperature sufficient to initiate copolymerization. The temperature at which copolymerization is initiated is preferably in the range of 70 - 200 °C, more preferably in the range of 70 - 150°C, and most preferably in the range of 70 - 120 °C.

In specific embodiments of the process for manufacturing a polymerization product, the process comprises the steps of:

a.) providing a polymerizable composition according to claims 3 or 4, comprising crosslinkable benzoxazines and cyclic carbonates,

b.) precuring the polymerizable composition by crosslinking the crosslinkable benzoxazines, and

c.) heating the precured polymerizable composition to a temperature sufficient to initiate copolymerization of the crosslinked benzoxazines and cyclic carbonates.

Thus, as described above, step b.) precuring of the expandable, polymerizable composition may be performed by UV-mediated crosslinking of the crosslinkable benzoxazine monomers comprising available alkene and thiol functional groups via thiol-ene reactions prior to the copolymerization step c.), in which the expansion of the poly(benzoxazine)-co-poly(cyclic carbonate) takes place. In copolymerization step c.) heat at a temperature sufficient to initiate copolymerization is applied. The temperature at which copolymerization is initiated is preferably in the range of 70- 200 °C, more preferably in the range of 70 - 150 °C, and most preferably in the range of 70 - 120 °C. This two-step process comprising a precuring step b.) followed by a copolymerization/expansion step c.) is particularly useful in applications in the building and casting industry, since it allows for an easy and convenient handling/ arrangement of the precured, thus preformed, but not yet expanded, composition. For example, the precured composition that has already a desired shape may be placed in an opening to be sealed; in a next step, the application of heat initiates copolymerization and expansion of the resulting crosslinked poly(benzoxazine)-co-poly(cyclic carbonate).

The present invention is further demonstrated and illustrated by the following examples, yet without being restricted thereto.

Fig. 1 of chapter 3.1 below illustrates a thiol-ene precured but not copolymerized specimen (on the right) and a thiol-ene precured and copolymerized specimen (on the left), wherein both specimen are prepared with an expandable, polymerizable composition according to the invention.

Fig. 2 shows the expansion rate in [%] as a function of the concentration of the cyclic carbonate in the copolymer of Cl[p($B_1$+RD)$_{0\text{-}100}$-*stat*-pEC$_{0\text{-}100}$]; see chapter 3.B.1 below.

Fig. 3 shows the expansion rate in [%] as a function of the concentration of the cyclic carbonate in the copolymer Cl[p($B_1$+RD) $_{0\text{-}100}$-*stat*-pPC$_{0\text{-}100}$]; see chapter 3.B.2. below.

Fig. 4 shows the expansion rate in [%] as a function of the concentration of the cyclic carbonate in the copolymer Cl[p($B_2$+RD) $_{0\text{-}100}$-*stat*-pEC$_{0\text{-}100}$]; see chapter 3.B.3. below.

Fig. 5 shows the expansion rate in [%] as a function of the concentration of the cyclic carbonate in the copolymer Cl[p($B_2$+RD) $_{0\text{-}100}$-*stat*-pPC$_{0\text{-}100}$]; see chapter 3.B.4. below.

Fig. 6 shows the expansion rate in [%] as a function of the concentration of the cyclic carbonate in the copolymer Cl[p($B_3$+RD) $_{0\text{-}100}$-*stat*-pEC$_{0\text{-}100}$]; see chapter 3.B.5. below.

Fig. 7 shows the expansion rate in [%] as a function of the concentration of the cyclic carbonate in the copolymer Cl[p($B_3$+RD) $_{0\text{-}100}$-*stat*-pPC$_{0\text{-}100}$]; see chapter 3.B.6. below.

Fig. 8 shows the expansion rate in [%] as a function of the concentration of the cyclic carbonate in the copolymer Cl[p($B_4$+RD) $_{0\text{-}100}$-*stat*-pEC$_{0\text{-}100}$]; see chapter 3.B.7. below.

Fig. 9 shows the expansion rate in [%] as a function of the concentration of the cyclic carbonate in the copolymer

Cl[p($B_4$+RD) $_{0-100}$-*stat*-pPC$_{0-100}$]; see chapter 3.B.8. below.

EXPERIMENTAL DATA and EXAMPLES

## 1. A. Materials and Methods

### 1.A.1 Materials

Bisphenol A (97%), hydroquinone (97%), para-formaldehyde (95%), phenol (>99%), m-cresol (>98%), 4-octylphenol (99%), 4-nonylphenol (>90%), sodium sulfate (>99%), ethylene carbonate (98%), propylene carbonate (99.7%), and diethyl ether (>98%) were purchased from Sigma-Aldrich Chemie GmbH (Vienna, Austria). Bisphenol F (>99%), Bisphenol S (>98%), pentaerythritol tetrakis(3-mercaptopropionate) (>90%) were purchased from TCI (Tokyo Chemical Industry Co., Ltd.; Austria, Vienna). Allylamine (>98%) was purchased from Thermo Fisher GmbH (Karlsruhe, Germany). Ethyl 2,4,6-trimethylbenzoylphenylphosphinate (>95%) was purchased from abcr GmbH (Karlsruhe, Germany). All substances were used without further purification.

### 1.A.2 Methods

Measurement of expansion rates - Density measurements

Density measurements were performed using a Mettler Toledo density measurement kit. The sample density is calculated with a hydrostatic balance in water, determining the uplift of specimens in and out of the solvent.

Expansion rates were quantified by density measurements of the monomers/precursors on the one hand and the expanded polymers on the other. Expansion rates were calculated according to the following formula:

$$\Delta V(\%) = \frac{\delta\,(\text{Mono.}) - \delta\,(\text{Polym.})}{\delta\,(\text{Polym.})} \times 100$$

FTIR, $^1$H-NMR and $^{13}$C-NMR measurements

FTIR-ATR spectra were recorded on a Bruker Alpha P instrument equipped with a DTGS detector (spectral range between 4000 and 800 cm$^{-1}$). The ATR unit is equipped with a diamond crystal. FTIR spectra were measured in transmission mode and obtained from powdered samples or liquid films.

NMR spectra were recorded on a Bruker Ultrashield 300 WB 300 MHz spectrometer. The solvent peak of $CDCl_3$ served as reference of the spectra (7.26 ppm for $^1$H and 77.0 ppm for $^{13}$C). The solvent residual peak of DMSO was used for referencing the spectra to 2.50 ($^1$H) and 39.5 ($^{13}$C) ppm. Peak shapes are indicated as follows: s (singlet), d (doublet), t (triplet), m (multiplet).

## 2.A. Monomer Synthesis

### 2.A.1 6,6'-(propane-2,2-diyl)bis(3-allyl-3,4-dihydro-2H-benzo[e][1,3loxazine) (acronym: $B_1$):

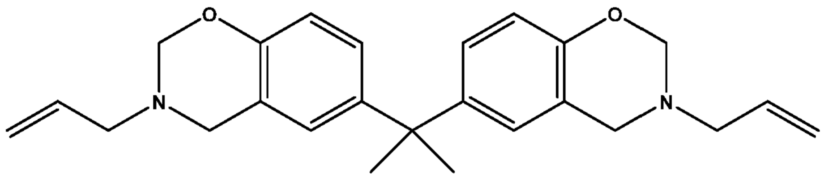

To a mixture of Bisphenol A (0.15 mol, 34.3 g) and allylamine (0.30 mol, 17.1 g), *para*-formaldehyde (0.62 mol, 18.6 g) is added in small portions over a period of 30 min, while being cooled in an ice bath to keep the temperature below 10 °C. Then, para-toluene sulfonic acid (2.90 mmol, 0.50 g) is added, the temperature is raised to 90 °C, and the mixture is stirred for 3 h. The resulting reaction mixture is dissolved in 500 mL of diethyl ether. The ether solution is washed with water (3 portions of 500 mL) to eliminate any unreacted formaldehyde, Bisphenol A or allyl amine and dried over sodium sulfate. The solvent is evaporated under reduced pressure and the product dried in a vacuum oven.

$^1$H-NMR (300 MHz, $CDCl_3$): δ (ppm) = 6.86 (2 H, m), 6.72 (2 H, m), 6.62 (2 H, m), 5.80 (2 H, m), 5.12 (4 H, m), 4.74 (4 H, s), 3.85 (4 H, s), 3.31 (2 H, d), 1.51 (6 H, s).
$^{13}$C-NMR (75 MHz, $CDCl_3$): δ (ppm) = 151.9, 143.8, 135.1, 126.3, 122.5, 119.2, 118.3, 115.8, 81.9, 54.5, 50.3, 41.7, 31.1.
FTIR (ATR): ν ($cm^{-1}$) = 3074, 2964, 2894, 2825, 1611, 1495, 1330, 1226, 1187, 1115, 987, 928, 814.

2.A.2 Bis(3-allyl-3,4-dihydro-2H-benzo[e][1,3]oxazin-6-yl)methane **(acronym: $B_2$):**

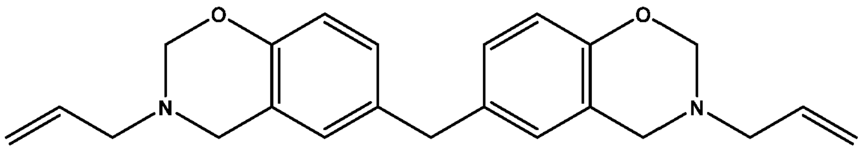

To a mixture of Bisphenol F (0.15 mol, 29.7 g) and allylamine (0.30 mol, 17.1 g), *para*-formaldehyde (0.62 mol, 18.6 g) is added in small portions over a period of 30 min, while being cooled in an ice bath to keep the temperature below 10 °C. Then, *para*-toluene sulfonic acid (2.90 mmol, 0.50 g) is added, the temperature is raised to 90 °C, and the mixture is stirred for 3 h. The resulting reaction mixture is then dissolved in 500 mL of diethyl ether. The ether solution is washed with water (3 portions of 500 mL) to eliminate any unreacted *para*-formaldehyde, Bisphenol F or allyl amine, and dried over sodium sulfate. The solvent is evaporated under reduced pressure and the product is dried in a vacuum oven.
$^1$H-NMR (300 MHz, $CDCl_3$): δ (ppm) = 6.83 (2 H, m), 6.67 (2 H, m), 6.60 (2 H, m), 5.79 (2 H, m), 5.10 (4 H, m), 4.73 (4 H, s), 3.84 (4 H, s), 3.66 (2 H, s), 3.30 (2 H, d).
$^{13}$C-NMR (75 MHz, $CDCl_3$): δ (ppm) = 152.5, 135.3, 133.7, 128.2, 127.7, 120.1, 118.4, 116.5, 82.5, 55.6, 49.8, 40.5.
FTIR (ATR): ν ($cm^{-1}$) = 3074, 2972, 2917, 2863, 1740, 1619, 1493, 1436, 1368, 1211, 1109, 987, 928, 811.

**2.A.3 6,6'-sulfonylbis(3-allyl-3,4-dihydro-2H-benzo[e][1,3]oxazine) (acronym: $B_3$):**

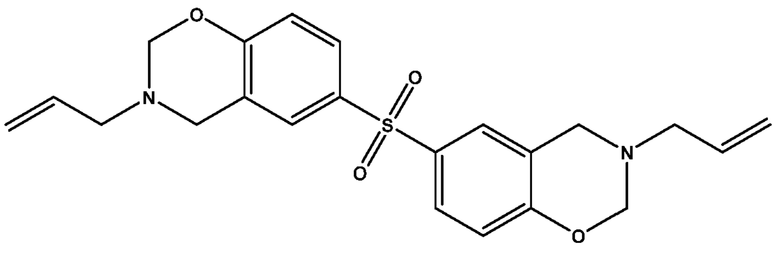

To a mixture of *para*-formaldehyde (0.62 mol, 18.6 g) and allylamine (0.30 mol, 17.1 g), Bisphenol S (0.15 mol, 39.3 g) is added in small portions over a period of 30 min, while being stirred at 60°C. Then, *para*-toluene sulfonic acid (2.90 mmol, 0.50 g) is added, the temperature is raised to 90 °C, and the mixture stirred for 3 h. The resulting reaction mixture is then dissolved in 500 mL of chloroform. The solution is washed with water (3 portions of 500 mL) to eliminate any unreacted *para*-formaldehyde, Bisphenol S or allyl amine and dried over sodium sulfate. The solvent is evaporated under reduced pressure, and the product is dried in a vacuum oven.
$^1$H-NMR (300 MHz, $CDCl_3$): δ (ppm) = 7.83 (2 H, m), 7.68 (2 H, m), 5,90 (2 H, m), 5.21 (4 H, m), 4.81 (4 H, s), 4.07 (4 H, s), 3.32 (4 H, d).
$^{13}$C-NMR (75 MHz, DMSO): δ (ppm) = 163.4, 135.4, 134.9, 130.4, 128.2, 122.2, 118.5, 117.2, 55.1, 49.8.
FTIR (ATR): ν ($cm^{-1}$) = 3072, 2958, 2007, 2850, 1683, 1577, 1484, 1440, 1287, 1442, 1081, 991, 916, 822.

**2.A.4 2,9-diallyl-1,2,3,8,9,10-hexahydrobenzo[2,1-e:3,4-e']bis([1,3]oxazine) (acronym: $B_4$):**

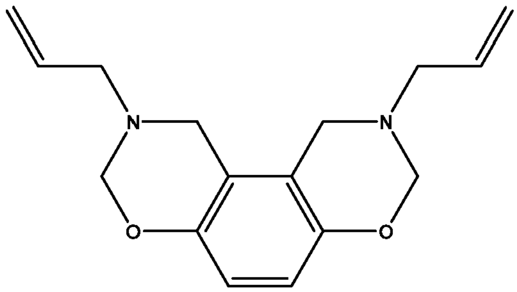

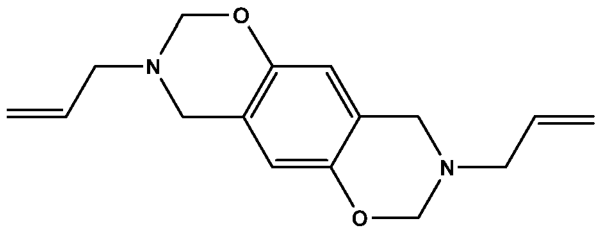

To a mixture of hydroquinone (0.15 mol, 16.5 g) and allylamine (0.30 mol, 17.1 g), *para*-formaldehyde (0.62 mol, 18.6 g) is added in small portions over a period of 30 min, while being cooled in an ice bath to keep the temperature below 10 °C. Then, *para*-toluene sulfonic acid (2.90 mmol, 0.50 g) is added, the temperature is raised to 90 °C, and the mixture is stirred for 3 h. The resulting brown reaction mixture is dissolved in 500 mL of diethyl ether. The ether solution is washed with water (3 portions of 500 mL) to eliminate any unreacted *para*-formaldehyde, hydroquinone or allyl amine and dried over sodium sulfate. The solvent is evaporated under reduced pressure and the product is dried in a vacuum oven.

$^{1}$H-NMR (300 MHz, $CDCl_3$): δ (ppm) = 6.61 (2 H, m), 6.43 (2 H, m), 6.92 (2 H, d), 5.87 (2 H, m), 5.18 (4 H, m), 4.92 (4 H, s), 4.75 (4 H, s), 3.78 (4 H, s), 3.68 (4 H, s), 3.36 (4 H, d), 3.14 (4 H, d).

$^{13}$C-NMR (75 MHz, DMSO): δ (ppm) = 150.3, 147.7, 135.0, 118.4, 117.9, 117.5, 117.0, 116.4, 115.6,115.0,114.4,113.0, 81.6, 67.1, 55.8, 54.5,49.7,46.6.

FTIR (ATR): ν ($cm^{-1}$) = 3076, 2970, 2850, 1740, 1475, 1366, 1230, 1117, 985, 916, 805.

**2.A.5 3-allyl-3,4-dihydro-2H-benzo[e][1,3]oxazine (reactive diluent) (acronym: RD):**

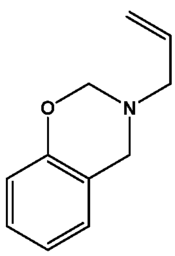

To a mixture of phenol (0.30 mol, 28.2 g) and allylamine (0.30 mol, 17.1 g), *para*-formaldehyde (0.62 mol, 18.6 g) is added in small portions over a period of 30 min, while being cooled in an ice bath to keep the temperature below 10 °C. Then, *para*-toluene sulfonic acid (2.90 mmol, 0.50 g) is added, the temperature is raised to 90 °C, and the mixture is stirred for 3 h. The resulting brown reaction mixture is then dissolved in 500 mL of diethyl ether. The ether solution is washed with water (3 portions of 500 mL) to eliminate any unreacted *para*-formaldehyde, phenol or allyl amine and dried over sodium sulfate. The solvent is evaporated under reduced pressure and the product is dried in a vacuum oven.

$^{1}$H-NMR (300 MHz, $CDCl_3$): δ (ppm) = 7.02 (1 H, m), 6.87 (1 H, m), 6.78 (1 H, m), 6.69 (1 H, m), 5.81 (1 H, m), 5.14 (2 H, m), 4.79 (2 H, s), 3.91 (2 H, s), 3.30 (2 H, d).

$^{13}$C-NMR (75 MHz, $CDCl_3$): δ (ppm) = 158.1, 134.4, 130.1, 129.7, 127.6, 121.0, 117.2, 111.9, 85.4, 58.5,56.9.

FTIR (ATR): ν ($cm^{-1}$) = 3072, 2970, 2841, 1740, 1576, 1487, 1364, 1219, 1107, 991, 922, 850, 754.

**2.A.6 3-allyl-5-methyl-3,4-dihydro-2H-benzo[e][1,3]oxazine (reactive diluent)**

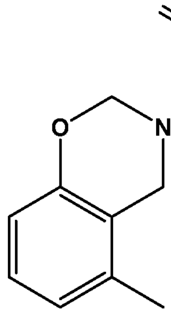

(acronym: RD-A):

To a mixture of m-cresol (0.30 mol, 32.4 g) and allylamine (0.30 mol, 17.1 g), *para*-formaldehyde (0.62 mol, 18.6 g) is added in small portions over a period of 30 min, while being cooled in an ice bath to keep the temperature below 10 °C. Then, *para*-toluene sulfonic acid (2.90 mmol, 0.50 g) is added, the temperature is raised to 90 °C, and the mixture is stirred for 3 h. The resulting brown reaction mixture is then dissolved in 500 mL of diethyl ether. The ether solution is washed with water (3 portions of 500 mL) to eliminate any unreacted formaldehyde, *m*-cresol or allyl amine and dried over sodium sulfate. The solvent is evaporated under reduced pressure and the product is dried in a vacuum oven.

$^{1}$H-NMR (300 MHz, $CDCl_3$): δ (ppm) = 6.72 (1 H, m), 6.64 (1 H, m), 6.53 (1 H, m), 5.82 (1 H, m), 5.10 (2 H, m), 4.81 (2 H, s), 3.86 (2 H, s), 3.08 (2 H, d), 2.17 (3 H, s).

$^{13}$C-NMR (75 MHz, $CDCl_3$): δ (ppm) = 157.9, 133.4, 130.2, 127.4, 122.2, 121.5, 118.3, 114.2, 82.1, 56.3, 49.4, 21.2.

FTIR (ATR): ν ($cm^{-1}$) = 2956, 2843, 1615, 1578, 1505, 1445, 1420, 1279, 1241, 1109, 990, 921, 860.

**2.A.7 3-allyl-6-octyl-3,4-dihydro-2H-benzo[e][1,3]oxazine (reactive diluent)**

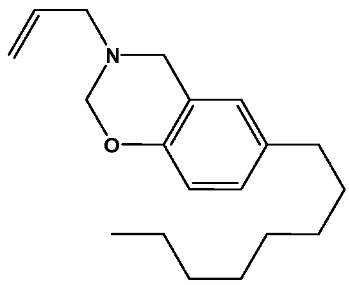

(acronym: RD-B):

To a mixture of 4-octylphenol (0.30 mol, 61.8 g) and allylamine (0.30 mol, 17.1 g), *para*-formaldehyde (0.62 mol, 18.6 g) is added in small portions over a period of 30 min, while being cooled in an ice bath to keep the temperature below 10 °C. Then, *para*-toluene sulfonic acid (2.90 mmol, 0.50 g) is added, the temperature is raised to 90 °C, and the mixture is stirred for 3 h. The resulting brown reaction mixture is then dissolved in 500 mL of diethyl ether. The ether solution is washed with water (3 portions of 500 mL) to eliminate any unreacted *para*-formaldehyde, 4-octylphenol or allyl amine and dried over sodium sulfate. The solvent is evaporated under reduced pressure and the product is dried in a vacuum oven.

$^{1}$H-NMR (300 MHz, $CDCl_3$): δ (ppm) = 6.81 (1 H, d, $^{3}J_{H,H}$ = 6.65 Hz), 6.64 (1 H, s), 6.60 (1 H, $^{3}J_{H,H}$ = 6.65 Hz), 5.85 (1 H, m), 5.12 (2 H, m), 4.84 (2 H, s), 3.88 (2 H, s), 3.21 (2 H, d), 2.41 (2 H, t), 1.48 (2 H, m), 1.19 (10 H, m), 0.79 (2 H, m).

$^{13}$C-NMR (75 MHz, $CDCl_3$): δ (ppm) = 152.0, 135.3, 135.1, 127.6, 127.2, 118.1, 116.2, 82.0, 54.6, 49.7, 35.3, 31.9, 31.7, 29.5, 29.4, 29.3, 22.7, 14.1.

FTIR (ATR): ν ($cm^{-1}$) = 2923, 2852, 1499, 1217, 1117, 988, 919, 820.

**2.A.8 3-allyl-6-nonyl-3,4-dihydro-2H-benzo[e][1,3]oxazine (reactive diluent)**

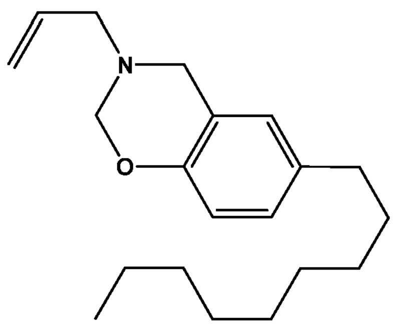

(acronym: RD-C):

To a mixture of 4-nonylphenol (0.30 mol, 66.1 g) and allylamine (0.30 mol, 17.1 g), *para*-formaldehyde (0.62 mol, 18.6 g) is added in small portions over a period of 30 min, while being cooled in an ice bath to keep the temperature below 10 °C. Then, *para*-toluene sulfonic acid (2.90 mmol, 0.50 g) is added, the temperature is raised to 90 °C, and the mixture is stirred for 3 h. The resulting brown reaction mixture is then dissolved in 500 mL of diethyl ether. The ether solution is washed with water (3 portions of 500 mL) to eliminate any unreacted *para*-formaldehyde, 4-nonylphenol or allyl amine and dried over sodium sulfate. The solvent is evaporated under reduced pressure and the product is dried in a vacuum oven.

$^1$H-NMR (300 MHz, $CDCl_3$): $\delta$ (ppm) = 6.89 (1 H, d), 6.82 (1 H, s), 6.55 (1 H,), 5.76 (1 H, m), 5.01 (2 H, m), 4.66 (2 H, s), 3.85 (2 H, s), 3.21 (2 H, d), 2.99 (2 H, m), 1.91 (2 H, m), 1.13 (2 H, m), 1.08 (2 H, m), 0.99 (4 H, m), 0.67 (4 H, m), 0.53 (3 H, m).

$^{13}$C-NMR (75 MHz, $CDCl_3$): $\delta$ (ppm) = 136.7, 126.6, 125.9, 125.7, 119.2, 118.0, 116.4, 82.8, 57.3, 53.0, 36.4, 32.0, 31.5, 29.8, 29.4, 29.1, 22.5, 14.6.

FTIR (ATR): ν ($cm^{-1}$) = 2957, 2851, 1500, 1378, 1230, 1122, 988, 822, 820, 748.

## 2.B. Synthesis of Homopolymers (comparative examples)

### 2.B1. Homopolymerization of Crosslinked Benzoxazines (comparative examples)

The acronym for the crosslinked (Cl) copolymers of the benzoxazine **$B_n$** and the reactive diluent **RD** is composed as follows: Cl[p($B_n$+RD)$_{100}$].

As tetrathiol **4SH,** pentaerythritol tetrakis(3-mercaptopropionate) is used.

As photoinitiator **PI,** ethyl(2,4,6-trimethylbenzoyl)phenylphosphinic ethyl ester is used.

#### 2.B1.1. Example 1 (comparative): Crosslinked copolymers of 6,6'-(propane-2,2-diyl)bis(3-allyl-3,4-dihydro-2H-benzo[e][1,3]oxazine) and 3-allyl-3,4-dihydro-2H-benzo[e] [1,3]oxazine

**(acronym: Cl[p($B_1$+RD)$_{100}$]):**

Boron trifluoride etherate (1.10 mmol, 156 mg) is added to **4SH** (3.1 mmol, 1.51 g). The mixture is added to a liquid blend of **$B_1$** (8.1 mmol, 3.16 g), **RD** (13.8 mmol, 2.41 g) and **PI** (0.1 mmol, 31.6 mg). Using UV-light, thiol-ene precuring is performed for 15 min. Subsequent heating up to 100 °C for 24 h results in the cationic ring-opening polymerization of **$B_1$** and **RD.**

FTIR (ATR): ν ($cm^{-1}$) = 3011, 2968, 2919, 1740, 1625, 1438, 1364, 1215, 1117, 911.

#### 2.B1.2. Example 2 (comparative) : Crosslinked copolymers of bis(3-allyl-3,4-dihydro-2H-benzo[e][1,3]oxazin-6-yl)methane and 3-allyl-3,4-dihydro-2H-benzo[e] [1,3]oxazine (acronym: Cl[p($B_2$+RD)$_{100}$]):

Boron trifluoride etherate (1.10 mmol, 156.1 mg) is added to **4SH** (3.1 mmol, 1.51 g). The mixture is added to a liquid blend of **$B_2$** (8.1 mmol, 2.94 g), **RD** (10.3 mmol, 1.80 g) and PI (0.1 mmol, 31.6 mg). Using UV-light, thiol-ene precuring is performed for 15 min. Subsequent heating up to 100 °C for 24 h results in the cationic ring-opening polymerization of **$B_2$** and **RD.**

FTIR (ATR): ν ($cm^{-1}$) = 3017, 2968, 2860, 1740, 1440, 1368, 1226, 1054, 869.

**2.B1.3. Example 3 (comparative): Crosslinked copolymers of 6,6'-sulfonylbis(3-allyl-3,4-dihydro-2H-benzo[e][1,3]oxazine) and 3-allyl-3,4-dihydro-2H-benzo[e][1,3]-oxazine**

**(acronym: Cl[p($B_3$+RD)$_{100}$]):**

Boron trifluoride etherate (1.10 mmol, 156.1 mg) is added to **4SH** (3.1 mmol, 1.51 g). The mixture is added to a liquid blend of **$B_3$** (8.1 mmol, 3.34 g), **RD** (10.3 mmol, 1.80 g) and **PI** (0.1 mmol, 31.6 mg). Using UV-light, thiol-ene precuring is performed for 15 min. Subsequent heating up to 100 °C for 24 h results in the cationic ring-opening polymerization of **$B_3$** and **RD.**

FTIR (ATR): ν ($cm^{-1}$) = 2921, 2853, 1683,1 578, 1458, 1290, 1098, 911·

**2.B1.4. Example 4 (comparative): Crosslinked copolymers of 2,9-diallyl-1,2,3,8,9,10-hexahydrobenzo[2,1-e:3,4-e']bis([1,3]oxazine and 3-allyl-3,4-dihydro-2H-benzo[e][1,3]oxazine**

**(acronym: Cl[p($B_4$+RD)$_{100}$]):**

Boron trifluoride etherate (1.10 mmol, 156.1 mg) is added to **4SH** (3.1 mmol, 1.51 g). The mixture is added to a liquid blend of **$B_4$** (12.1 mmol, 3.29 g), **RD** (9.8 mmol, 2.66 g) and **PI** (0.1 mmol, 31.6 mg). Using UV-light, thiol-ene precuring is performed for 15 min. Subsequent heating up to 100 °C for 24 h results in the cationic ring-opening polymerization of **$B_4$** and **RD.**

FTIR (ATR): ν ($cm^{-1}$) = 3017, 2970, 1740, 1640, 1444, 1368, 1219, 911.

**2.B2. Homopolymerization of Cyclic Carbonates (comparative examples)**

**2.B2.1. Example 5 (comparative): Polyethylene carbonate) (acronym: pEC):**

Boron trifluoride etherate (1.86 mmol, 265 mg) is added to molten ethylene carbonate (EC) (0.10 mol, 8.8 g). The reaction mixture is heated up to 100 °C overnight without stirring. After 24 h, a solid polymer is obtained.

FTIR (ATR): ν ($cm^{-1}$) = 3007, 2970, 2878, 1744, 1442, 1364, 1219, 1048, 879

**2.B2.2. Example 6 (comparative): Poly(propylene carbonate)**

**(acronym: pPC):**

Boron trifluoride etherate (1.9 mmol, 265 mg) is added to propylene carbonate (PC) (0.10 mol, 10.2 g). The reaction mixture is heated up to 100 °C overnight without stirring. After 24 h, a solid polymer is obtained.

FTIR (ATR): ν ($cm^{-1}$) = 2988, 2923, 1789, 1485, 1379, 1180, 1040, 885, 773.

**2.C. Copolymer Synthesis (Examples according to the invention)**

**2.C.1. Example 7 (invention): Cl[p($B_1$+RD)$_i$-*stat*-pEC$_{100-i}$] (i = 99, 97, 93, 86, 70, 30, 1) & [p($B_1$+RD)$_i$-*stat*-pEC$_{100-i}$] (i = 86)**

Boron trifluoride etherate (amount: A) is added to **4SH** (amount: B). The mixture is added to a liquid blend of **$B_1$** (amount: C), **RD** (amount: D), **EC** (amount: E) and **PI** (amount: F) - see Table 1 below. Using UV-light, thiol-ene precuring is performed for 15 min. Subsequent heating up to 100 °C for 24 h results in the cationic ring-opening copolymerization of **$B_1$**, **RD** and **EC.** In the case of [p($B_1$+RD)$_{86}$-*stat*-pEC$_{14}$], neither **4SH** nor **PI** were added; no UV illumination was applied.

FTIR (ATR): ν ($cm^{-1}$) = 3074, 2964, 2825, 1809, 1593, 1493, 1344, 1222, 1120, 1073, 922, 816, 754.

Table 1:

| Identifier | A [mmol//g] | B [mmol//g] | C [mmol//g] | D [mmol//g] | E [mmol//g] | F [mmol//g] |
|---|---|---|---|---|---|---|
| Cl[p($B_1$+RD)$_{99}$-stat-pEC$_1$] | 1.10// 0.156 | 3.1//1.51 | 8.1//3.16 | 13.8//2.41 | 0.83//0.073 | 0.10// 0.031 |
| Cl[p($B_1$+RD)$_{97}$-stat-pEC$_3$] | 1.10// 0.156 | 3.1//1.51 | 8.1//3.16 | 13.8//2.41 | 2.55//0.225 | 0.10// 0.031 |

(continued)

| Identifier | A [mmol//g] | B [mmol//g] | C [mmol//g] | D [mmol//g] | E [mmol//g] | F [mmol//g] |
|---|---|---|---|---|---|---|
| $Cl[p(B_1+RD)_{93}$-stat-$pEC_7]$ | 1.10// 0.156 | 3.1//1.51 | 8.1//3.16 | 13.8//2.41 | 6.61//0.550 | 0.10// 0.031 |
| $Cl[p(B_1+RD)_{86}$-stat-$pEC_{14}]$ | 1.10// 0.156 | 3.1//1.51 | 8.1//3.16 | 13.8//2.41 | 15.7//1.38 | 0.10// 0.031 |
| $Cl[p(B_1+RD)_{70}$-stat-$pEC_{30}]$ | 1.10// 0.156 | 3.1//1.51 | 8.1//3.16 | 13.8//2.41 | 38.5//3.11 | 0.10// 0.031 |
| $Cl[p(B_1+RD)_{30}$-stat-$pEC_{70}]$ | 0.20// 0.029 | 0.57// 0.280 | 1.48//0.58 | 2.52//0.44 | 38.5//3.11 | 0.018// 0.0060 |
| $Cl[p(B_1+RD)_1$-stat-$pEC_{99}]$ | 0.03// 0.0042 | 0.084// 0.041 | 0.22//0.085 | 0.37//0.065 | 231//18.66 | 0.003// 0.0011 |
| $[p(B_1+RD)_{86}$-*stat*-$pEC_{14}]$ | 1.10// 0.156 | 0//0 | 8.1//3.16 | 13.8//2.41 | 15.7//1.38 | 0//0 |

**2.C.2. Example 8 (invention): $Cl[p(B_1+RD)_i$-*stat*-$pPC_{100-i}]$ (i = 99, 97, 93, 86, 70, 30, 1) & $[p(B_1+RD)_i$-*stat*-$pPC_{100-i}]$ (i = 86)**

Boron trifluoride etherate (amount: A) is added to **4SH** (amount: B). The mixture is added to a liquid blend of $\mathbf{B_1}$ (amount: C), **RD** (amount: D), **PC** (amount: E) and **PI** (amount: F) - see Table 2 below. Using UV-light, thiol-ene precuring is performed for 15 min. Subsequent heating up to 100 °C for 24 h results in the cationic ring-opening copolymerization of $\mathbf{B_1}$, **RD,** and **PC.** In the case of $[p(B_1+RD)_{86}$-*stat*-$pPC_{14}]$, neither **4SH** nor **PI** were added; no UV illumination was applied.

FTIR (ATR): ν ($cm^{-1}$) = 2968, 2925, 2821, 1740, 1591, 1493, 1366, 1236, 1132, 987, 911, 820, 754.

Table 2:

| Identifier | A [mmol//g] | B [mmol//g] | C [mmol//g] | D [mmol//g] | E [mmol//g] | F [mmol//g] |
|---|---|---|---|---|---|---|
| $Cl[p(B_1+RD)_{99}$-stat-$pPC_1]$ | 1.10// 0.156 | 3.1//1.51 | 8.1//3.16 | 13.8//2.41 | 0.72// 0.0734 | 0.100// 0.0310 |
| $Cl[p(B_1+RD)_{97}$-stat-$pPC_3]$ | 1.10// 0.156 | 3.1//1.51 | 8.1//3.16 | 13.8//2.41 | 2.2//0.225 | 0.100// 0.0310 |
| $Cl[p(B_1+RD)_{93}$-stat-$pPC_7]$ | 1.10// 0.156 | 3.1//1.51 | 8.1//3.16 | 13.8//2.41 | 5.4//0.55 | 0.100// 0.0310 |
| $Cl[p(B_1+RD)_{86}$-stat-$pPC_{14}]$ | 1.10// 0.156 | 3.1//1.51 | 8.1//3.16 | 13.8//2.41 | 13.5//1.38 | 0.100// 0.0310 |
| $Cl[p(B_1+RD)_{70}$-stat-$pPC_{30}]$ | 1.10// 0.156 | 3.1//1.51 | 8.1//3.16 | 13.8//2.41 | 30.5//3.11 | 0.100// 0.0310 |
| $Cl[p(B_1+RD)_{30}$-stat-$pPC_{70}]$ | 0.20// 0.0285 | 0.57// 0.280 | 1.48//0.578 | 2.52// 0.441 | 30.5//3.11 | 0.018// 0.0060 |
| $Cl[p(B_1+RD)_1$-stat-$pPC_{99}]$ | 0.03// 0.0042 | 0.084// 0.041 | 0.22//0.085 | 0.371// 0.065 | 183//18.66 | 0.003// 0.0011 |
| $[p(B_1+RD)_{86}$-*stat*-$pPC_{14}]$ | 1.10// 0.156 | 0//0 | 8.1//3.16 | 13.8//2.41 | 13.5//1.38 | 0//0 |

**2.C.3. Example 9 (invention): $Cl[p(B_2+RD)_i$-*stat*-$pEC_{100-i}]$ (i = 99, 97, 93, 86, 70, 30, 1) & $[p(B_2+RD)_i$-*stat*-$pEC_{100-i}]$ (i = 86)**

Boron trifluoride etherate (amount: A) is added to **4SH** (amount: B). The mixture is added to a liquid blend of $\mathbf{B_2}$ (amount: C), **RD** (amount: D), **EC** (amount: E) and **PI** (amount: F) - see Table 3 below. Using UV-light, thiol-ene precuring is performed for 15 min. Subsequent heating up to 100 °C for 24 h results in the cationic ring-opening polymerization of $\mathbf{B_2}$, **RD,** and EC. In the case of $[p(B_2+RD)_{86}$-*stat*-$pEC_{14}]$, neither **4SH** nor **PI** were added; no UV illumination was applied.

FTIR (ATR): ν ($cm^{-1}$) = 3005, 2968, 2926, 1736, 1594, 1438, 1364, 1230, 1236, 987, 920, 811.

Table 3:

| Identifier | A [mmol//g] | B [mmol//g] | C [mmol//g] | D [mmol//g] | E [mmol//g] | F [mmol//g] |
|---|---|---|---|---|---|---|
| Cl[p($B_2$+RD)$_{99}$-stat-pEC$_1$] | 1.10//0.156 | 3.1//1.51 | 8.1//2.94 | 10.3//1.80 | 0.74// 0.0652 | 0.100// 0.031 |
| Cl[p($B_2$+RD)$_{97}$-stat-pEC$_3$] | 1.10//0.156 | 3.1//1.51 | 8.1//2.94 | 10.3//1.80 | 2.26// 0.199 | 0.100// 0.031 |
| Cl[p($B_2$+RD)$_{93}$-stat-pEC$_7$] | 1.10//0.156 | 3.1//1.51 | 8.1//2.94 | 10.3//1.80 | 5.5//0.484 | 0.100// 0.031 |
| Cl[p($B_2$+RD)$_{86}$-stat-pEC$_{14}$] | 1.10//0.156 | 3.1//1.51 | 8.1//2.94 | 10.3//1.80 | 13.9//1.23 | 0.100// 0.031 |
| Cl[p($B_2$+RD)$_{70}$-stat-pEC$_{30}$] | 1.10//0.156 | 3.1//1.51 | 8.1//2.94 | 10.3//1.80 | 31.3//2.76 | 0.100// 0.031 |
| Cl[p($B_2$+RD)$_{30}$-stat-pEC$_{70}$] | 0.17// 0.0247 | 0.49//0.239 | 1.28//0.466 | 1.62// 0.285 | 31.3//2.76 | 0.015// 0.0016 |
| Cl[p($B_2$+RD)$_1$-stat-pEC$_{99}$] | 0.025// 0.0035 | 0.070// 0.0339 | 0.182// 0.0661 | 0.23// 0.0405 | 188//16.6 | 0.002// 0.0007 |
| [p($B_2$+RD)$_{86}$-*stat*-pEC$_{14}$] | 1.10//0.156 | 0//0 | 8.1//2.94 | 10.3//1.80 | 13.9//1.23 | 0//0 |

**2.C.4. <u>Example 10 (invention):</u> Cl[p($B_2$+RD)$_i$-*stat*-pPC$_{100-i}$] (i = 99, 97, 93, 86, 70, 30, 1) & [p($B_2$+RD)$_i$-*stat*-pPC$_{100-i}$] (i = 86)**

Boron trifluoride etherate (amount: A) is added to **4SH** (amount: B). The mixture is added to a liquid blend of **$B_2$** (amount: C), **RD** (amount: D), **PC** (amount: E) and **PI** (amount: F) - see Table 4 below. Using UV-light, thiol-ene precuring is performed for 15 min. Subsequent heating up to 100 °C for 24 h results in the cationic ring-opening polymerization of **$B_2$**, **RD,** and PC. In the case of [p($B_2$+RD)$_{86}$-*stat*-pPC$_{14}$], neither **4SH** nor **PI** were added; no UV illumination was applied.

FTIR (ATR): v (cm$^{-1}$) = 3070, 2923, 2839, 1797, 1736, 1591, 1493, 1354, 1246, 1117, 991, 926, 765.

Table 4:

| Identifier | A [mmol//g] | B [mmol//g] | C [mmol//g] | D [mmol//g] | E [mmol//g] | F [mmol//g] |
|---|---|---|---|---|---|---|
| Cl[p($B_2$+RD)$_{99}$-stat-pPC$_1$] | 1.10//0.156 | 3.1//1.51 | 8.1//2.94 | 10.3//1.80 | 0.64// 0.0652 | 0.100// 0.031 |
| Cl[p($B_2$+RD)$_{97}$-stat-pPC$_3$] | 1.10//0.156 | 3.1//1.51 | 8.1//2.94 | 10.3//1.80 | 1.95// 0.199 | 0.100// 0.031 |
| Cl[p($B_2$+RD)$_{93}$-stat-pPC$_7$] | 1.10//0.156 | 3.1//1.51 | 8.1//2.94 | 10.3//1.80 | 4.8//0.484 | 0.100// 0.031 |
| Cl[p($B_2$+RD)$_{86}$-stat-pPC$_{14}$] | 1.10//0.156 | 3.1//1.51 | 8.1//2.94 | 10.3//1.80 | 12//1.23 | 0.100// 0.031 |
| Cl[p($B_2$+RD)$_{70}$-stat-pPC$_{30}$] | 1.10//0.156 | 3.1//1.51 | 8.1//2.94 | 10.3//1.80 | 27//2.76 | 0.100// 0.031 |
| Cl[p($B_2$+RD)$_{30}$-stat-pPC$_{70}$] | 0.17// 0.0247 | 0.49//0.239 | 1.28//0.466 | 1.62// 0.285 | 27//2.76 | 0.015// 0.0016 |
| Cl[p($B_2$+RD)$_1$-stat-pPC$_{99}$] | 0.025// 0.0035 | 0.070// 0.0339 | 0.182// 0.0661 | 0.23// 0.0405 | 162//16.6 | 0.002// 0.0007 |
| [p($B_2$+RD)$_{86}$-*stat*-pPC$_{14}$] | 1.10//0.156 | 0//0 | 8.1//2.94 | 10.3//1.80 | 12//1.23 | 0//0 |

**2.C.5. <u>Example 11 (invention):</u> Cl[p($B_3$+RD)$_i$-*stat*-pEC$_{100-i}$] (i = 99, 97, 93, 86, 70, 30, 1)**

Boron trifluoride etherate (amount: A) is added to **4SH** (amount: B). The mixture is added to a liquid blend of **$B_3$** (amount: C), **RD** (amount: D), **EC** (amount: E) and **PI** (amount: F) - see Table 5 below. Using UV-light, thiol-ene precuring is performed for 15 min. Subsequent heating up to 100 °C for 24 h results in the cationic ring-opening polym-

erization of **$B_3$**, **RD,** and **EC.**

FTIR (ATR): ν ($cm^{-1}$) = 3004, 2919, 2815, 1729, 1592, 1452, 1252, 1136, 999, 920.

Table 5:

| Identifier | A [mmol//g] | B [mmol//g] | C [mmol//g] | D [mmol//g] | E [mmol//g] | F [mmol//g] |
|---|---|---|---|---|---|---|
| Cl[p($B_3$+RD)$_{99}$-stat-pEC$_1$] | 1.10//0.156 | 3.1//1.51 | 8.1//3.34 | 10.3//1.80 | 0.78//0.0691 | 0.100//0.0310 |
| Cl[p($B_3$+RD)$_{97}$-stat-pEC$_3$] | 1.10//0.156 | 3.1//1.51 | 8.1//3.34 | 10.3//1.80 | 2.4//0.212 | 0.100//0.0310 |
| Cl[p($B_3$+RD)$_{93}$-stat-pEC$_7$] | 1.10//0.156 | 3.1//1.51 | 8.1//3.34 | 10.3//1.80 | 5.9//0.515 | 0.100//0.0310 |
| Cl[p($B_3$+RD)$_{86}$-stat-pEC$_{14}$] | 1.10//0.156 | 3.1//1.51 | 8.1//3.34 | 10.3//1.80 | 12.6/1.11 | 0.100//0.0310 |
| Cl[p($B_3$+RD)$_{70}$-stat-pEC$_{30}$] | 1.10//0.156 | 3.1//1.51 | 8.1//3.34 | 10.3//1.80 | 33.3//2.93 | 0.100//0.0310 |
| Cl[p($B_3$+RD)$_{30}$-stat-pEC$_{70}$] | 0.19//0.0270 | 0.54//0.261 | 1.40//0.576 | 1.8//0.311 | 33.3//2.93 | 0.017//0.0054 |
| Cl[p($B_3$+RD)$_1$-stat-pEC$_{99}$] | 0.027//0.0038 | 0.076//0.0370 | 0.198//0.0818 | 0.25//0.0447 | 188//16.6 | 0.002//0.0008 |

**2.C.6. <u>Example 12 (invention):</u> Cl[p($B_3$+RD)$_i$-*stat*-pPC$_{100-i}$] (i = 99, 97, 93, 86, 70, 30, 1)**

Boron trifluoride etherate (amount: A) is added to **4SH** (amount: B). The mixture is added to a liquid blend of **$B_3$** (amount: C), **RD** (amount: D), **PC** (amount: E) and **PI** (amount: F) - see Table 6 below. Using UV-light, thiol-ene precuring is performed for 15 min. Subsequent heating up to 100 °C for 24 h results in the cationic ring-opening polymerization of **$B_3$**, **RD,** and **PC.**

FTIR (ATR): ν ($cm^{-1}$) = 3076, 2925, 2815,1730,1595,1460,1256,1142, 971, 916, 756.

Table 6:

| Identifier | A [mmol//g] | B [mmol//g] | C [mmol//g] | D [mmol// g] | E [mmol//g] | F [mmol//g] |
|---|---|---|---|---|---|---|
| Cl[p($B_3$+RD)$_{99}$-stat-pPC$_1$] | 1.10//0.156 | 3.1//1.51 | 8.1//3.34 | 10.3//1.80 | 0.68//0.0691 | 0.100//0.0310 |
| Cl[p($B_3$+RD)$_{97}$-stat-pPC$_3$] | 1.10//0.156 | 3.1//1.51 | 8.1//3.34 | 10.3//1.80 | 2.07//0.212 | 0.100//0.0310 |
| Cl[p($B_3$+RD)$_{93}$-stat-pPC$_7$] | 1.10//0.156 | 3.1//1.51 | 8.1//3.34 | 10.3//1.80 | 5.0//0.515 | 0.100//0.0310 |
| Cl[p($B_3$+RD)$_{86}$-stat-pPC$_{14}$] | 1.10//0.156 | 3.1//1.51 | 8.1//3.34 | 10.3//1.80 | 10.9/1.11 | 0.100//0.0310 |
| Cl[p($B_3$+RD)$_{70}$-stat-pPC$_{30}$] | 1.10//0.156 | 3.1//1.51 | 8.1//3.34 | 10.3//1.80 | 28.7//2.93 | 0.100//0.0310 |
| Cl[p($B_3$+RD)$_{30}$-stat-pPC$_{70}$] | 0.19//0.0270 | 0.54//0.261 | 1.40//0.577 | 1.77//0.311 | 28.7//2.93 | 0.017//0.0054 |

(continued)

| Identifier | A [mmol//g] | B [mmol//g] | C [mmol//g] | D [mmol// g] | E [mmol//g] | F [mmol//g] |
|---|---|---|---|---|---|---|
| Cl[p($B_3$+RD)$_1$-stat-pPC$_{99}$] | 0.027//0.0038 | 0.076//0.0370 | 0.198//0.0818 | 0.251// 0.441 | 162//16.6 | 0.002//0.0008 |

### 2.C.7. Example 13 (invention): Cl[p($B_4$+RD)$_i$-*stat*-pEC$_{100-i}$] (i = 99, 97, 93, 86, 70, 30, 1)

Boron trifluoride etherate (amount: A) is added to **4SH** (amount: B). The mixture is added to a liquid blend of $\mathbf{B_4}$ (amount: C), **RD** (amount: D), **EC** (amount: E) and **PI** (amount: F) - see Table 7 below. Using UV-light, thiol-ene precuring is performed for 15 min. Subsequent heating up to 100 °C for 24 h results in the cationic ring-opening polymerization of $\mathbf{B_4}$, **RD,** and **EC.**

FTIR (ATR): v (cm$^{-1}$) = 2998, 2917, 1762, 1638, 1622, 1479, 1391, 1156, 1052, 969, 716.

Table 7:

| Identifier | A [mmol//g] | B [mmol//g] | C [mmol//g] | D [mmol//g] | E [mmol//g] | F [mmol//g] |
|---|---|---|---|---|---|---|
| Cl[p($B_4$+RD)$_{99}$-stat-pEC$_1$] | 1.10//0.156 | 3.1//1.51 | 12.1//3.29 | 9.8//2.66 | 0.88// 0.0773 | 0.100// 0.0310 |
| Cl[p($B_4$+RD)$_{97}$-stat-pEC$_3$] | 1.10//0.156 | 3.1//1.51 | 12.1//3.29 | 9.8//2.66 | 2.69//0.237 | 0.100// 0.0310 |
| Cl[p($B_4$+RD)$_{93}$-stat-pEC$_7$] | 1.10//0.156 | 3.1//1.51 | 12.1//3.29 | 9.8//2.66 | 6.5//0.576 | 0.100// 0.0310 |
| Cl[p($B_4$+RD)$_{86}$-stat-pEC$_{14}$] | 1.10//0.156 | 3.1//1.51 | 12.1//3.29 | 9.8//2.66 | 16.5//1.46 | 0.100// 0.0310 |
| Cl[p($B_4$+RD)$_{70}$-stat-pEC$_{30}$] | 1.10//0.156 | 3.1//1.51 | 12.1//3.29 | 9.8//2.66 | 37.2//3.28 | 0.100// 0.0310 |
| Cl[p($B_4$+RD)$_{30}$-stat-pEC$_{70}$] | 0.203// 0.0288 | 0.57//0.278 | 2.23//0.607 | 1.80//0.491 | 37.2//3.28 | 0.018// 0.0057 |
| Cl[p($B_4$+RD)$_1$-stat-pEC$_{99}$] | 0.029// 0.0041 | 0.081// 0.0393 | 0.315// 0.0856 | 0.254// 0.0692 | 223//19.7 | 0.003// 0.0008 |

### 2.C.8. Example 14 (invention): Cl[p($B_4$+RD)$_i$-*stat*-pPC$_{100-i}$] (i = 99, 97, 93, 86, 70, 30, 1)

Boron trifluoride etherate (amount: A) is added to **4SH** (amount:B). The mixture is added to a liquid blend of $\mathbf{B_4}$ (amount: C), **RD** (amount: D), **PC** (amount: E) and **PI** (amount: F) - see Table 8 below. Using UV-light, thiol-ene precuring is performed for 15 min. Subsequent heating up to 100 °C for 24 h results in the cationic ring-opening polymerization of $\mathbf{B_4}$, **RD,** and **PC.**

FTIR (ATR): v (cm$^{-1}$) = 3070, 2928, 2854, 1740, 1640, 1617, 1436, 1354, 1217, 1130, 936.

Table 8:

| Identifier | A [mmol//g] | B [mmol//g] | C [mmol//g] | D [mmol//g] | E [mmol//g] | F [mmol//g] |
|---|---|---|---|---|---|---|
| Cl[p($B_4$+RD)$_{99}$-stat-pPC$_1$] | 1.10//0.156 | 3.1//1.51 | 12.1//3.29 | 9.8//2.66 | 0.76//0.0773 | 0.100// 0.0310 |
| Cl[p($B_4$+RD)$_{97}$-stat-pPC$_3$] | 1.10//0.156 | 3.1//1.51 | 12.1//3.29 | 9.8//2.66 | 2.32//0.237 | 0.100// 0.0310 |

(continued)

| Identifier | A [mmol//g] | B [mmol//g] | C [mmol//g] | D [mmol//g] | E [mmol//g] | F [mmol//g] |
|---|---|---|---|---|---|---|
| Cl[p $(B_4+RD)_{93}$-stat-$pPC_7$] | 1.10//0.156 | 3.1//1.51 | 12.1//3.29 | 9.8//2.66 | 5.6//0.576 | 0.100// 0.0310 |
| Cl[p $(B_4+RD)_{86}$-stat-$pPC_{14}$] | 1.10//0.156 | 3.1//1.51 | 12.1//3.29 | 9.8//2.66 | 14.3//1.46 | 0.100// 0.0310 |
| Cl[p $(B_4+RD)_{70}$-stat-$pPC_{30}$] | 1.10//0.156 | 3.1//1.51 | 12.1//3.29 | 9.8//2.66 | 32.1//3.28 | 0.100// 0.0310 |
| Cl[p $(B_4+RD)_{30}$-stat-$pPC_{70}$] | 0.203// 0.0288 | 0.57//0.278 | 2.23//0.607 | 1.80//0.491 | 32.1//3.28 | 0.018// 0.0057 |
| Cl[p $(B_4+RD)_1$-stat-$pPC_{99}$] | 0.029// 0.0041 | 0.081// 0.0393 | 0.32//0.0856 | 0.254// 0.0692 | 193//19.7 | 0.003// 0.0008 |

## 3. Expansion

### 3.A. Visual presentation of a copolymer according to the invention

In order to visually demonstrate the effect of the expansion upon curing, specimens were prepared as follows: Boron trifluoride etherate (1.1 mmol, 0.156 g) was added to **4SH** (3.1 mmol, 1.51 g). The mixture was added to a liquid blend of **$B_1$** (8.1 mmol, 3.16 g), RD (13.8 mmol, 2.41 g), **PC** (15.7 mmol, 1.38 g) and **PI** (0.10 mmol, 0.031 g) and poured into a circular-shaped mold. Using UV-light, thiol-ene precuring was performed for 15 min. The specimen was subsequently divided into two parts. One part was heated up to 100 °C for 24 h to enable cationic ring-opening copolymerization of **$B_1$**, **RD,** and **PC;** the other part was kept without additional curing.

Fig. 1 illustrates the thiol-ene precured but not copolymerized specimen (smaller part on the right) and the thiol-ene precured and copolymerized specimen (larger part on the left).

### 3.B. Expansion rates

Expansion rates were quantified by density measurements of the homopolymers (see above comparative examples 1 - 6) on the one hand and the compolymers according to the invention (see above examples 7 - 14 according to the invention) on the other. Expansion rates were measured and calculated as described above under chapter **1.A.2 Methods.** The expansion rates are given in Tables 10 - 17 below in chapters 3.B.1. to 3.B.8. and are further illustrated in accompanying Figs. 2 - 9.

While the comparative examples have been included within the individual tables in chapters 3.B.1. to 3.B.8., the NON-copolymerized congeners, namely the polybenzoxazine homopolymers on the one hand as well the poly(cyclic carbonate) homopolymers on the other, have been listed in Table 9 hereinafter in order to provide an overview of the expansion rates achievable according to the current state-of-the-art:

Table 9:

| Identifier | Expansion rate [%] |
|---|---|
| Cl[p$(B_1+RD)_{100}$] | 3.3 |
| Cl[p$(B_2+RD)_{100}$] | 2.1 |
| Cl[p$(B_3+RD)_{100}$] | 1.0 |
| Cl[p$(B_4+RD)_{100}$] | 0.2 |
| pEC | -0.8 |
| pPC | -0.5 |

**3.B.1. Cl[p($B_1$+RD)$_i$-*stat*-pEC$_{100-i}$] (i = 100, 99, 97, 93, 86, 70, 30, 1, 0) & [p($B_1$+RD)$_i$-*stat*-pEC$_{100-i}$] (i = 86)**

Table 10:

| Identifier | Appearance | $\delta_{Monomer}$ [g×cm$^{-1}$] | $\delta_{Polymer}$ [g×cm$^{-1}$] | Expansion rate [%] |
|---|---|---|---|---|
| Cl[p($B_1$+RD)$_{100}$] | solid, brittle | 1.169 | 1.132 | 3.3 |
| Cl[p($B_1$+RD)$_{99}$-stat-pEC$_1$] | solid, brittle | 1.174 | 1.129 | 4 |
| Cl[p($B_1$+RD)$_{97}$-stat-pEC$_3$] | solid, soft | 1.182 | 1.059 | 11.6 |
| Cl[p($B_1$+RD)$_{93}$-stat-pEC$_7$] | solid, soft | 1.117 | 0.989 | 12.9 |
| Cl[p($B_1$+RD)$_{86}$-stat-pEC$_{14}$] | rubbery | 1.143 | 0.938 | 21.9 |
| Cl[p($B_1$+RD)$_{70}$-stat-pEC$_{30}$] | rubbery | 1.273 | 0.962 | 32.4 |
| Cl[p($B_1$+RD)$_{30}$-stat-pEC$_{70}$] | rubbery, swollen | 1.217 | 1.182 | 2.9 |
| Cl[p($B_1$+RD)$_1$-stat-pEC$_{99}$] | liquid | 1.264 | 1.264 | 0 |
| pEC | liquid, highly viscous | 1.311 | 1.321 | -0.8 |
| [p($B_1$+RD)$_{86}$-*stat*-pEC$_{14}$] | rubbery | 1.187 | 1.014 | 17.1 |

Fig 2 shows the expansion rate in [%] as a function of the concentration of the cyclic carbonate in the copolymer Cl[p($B_1$+RD)$_{0-100}$-*stat*-pEC$_{0-100}$].

**3.B.2. Cl[p($B_1$+RD)$_i$-*stat*-pPC$_{100-i}$] (i = 100, 99, 97, 93, 86, 70, 30, 1, 0) & [p($B_1$+RD)$_i$-*stat*-pPc$_{100-i}$] (i = 86)**

Table 11:

| Identifier | Appearance | $\delta_{Monomer}$ [g×cm$^{-1}$] | $\delta_{Polymer}$ [g×cm$^{-1}$] | Expansion rate [%] |
|---|---|---|---|---|
| Cl[p($B_1$+RD)$_{100}$] | solid, brittle | 1.169 | 1.132 | 3.3 |
| Cl[p($B_1$+RD)$_{99}$-stat-pPC$_1$] | solid, brittle | 1.173 | 1.149 | 2.1 |
| Cl[p($B_1$+RD)$_{97}$-stat-pPC$_3$] | solid, soft | 1.184 | 1.089 | 8.8 |
| Cl[p($B_1$+RD)$_{93}$-stat-pPC$_7$] | solid, soft | 1.174 | 1.003 | 17.1 |
| Cl[p($B_1$+RD)$_{86}$-stat-pPC$_{14}$] | rubbery | 1.172 | 0.944 | 24.1 |
| Cl[p($B_1$+RD)$_{70}$-stat-pPC$_{30}$] | rubbery | 1.232 | 0.936 | 31.7 |
| Cl[p($B_1$+RD)$_{30}$-stat-pPC$_{70}$] | rubbery, swollen | 1.199 | 1.176 | 2 |
| Cl[p($B_1$+RD)$_1$-stat-pPC$_{99}$] | liquid | 1.226 | 1.225 | 0.1 |
| pPC | liquid, highly viscous | 1.229 | 1.235 | -0.5 |
| [p($B_1$+RD)$_{86}$-*stat*-pPC$_{14}$] | rubbery | 1.202 | 1.027 | 17 |

Fig. 3 shows the expansion rate in [%] as a function of the concentration of the cyclic carbonate in the copolymer Cl[p($B_1$+RD)$_{0-100}$-*stat*-pPC$_{0-100}$].

**3.B.3. Cl[p($B_2$+RD)$_i$-*stat*-pEC$_{100-i}$] (i = 100, 99, 97, 93, 86, 70, 30, 1, 0) & [p($B_2$+RD)$_i$-*stat*-pEC$_{100-i}$] (i = 86)**

Table 12:

| Identifier | Appearance | $\delta_{Monomer}$ [g×cm$^{-1}$] | $\delta_{Polymer}$ [g×cm$^{-1}$] | Expansion rate [%] |
|---|---|---|---|---|
| Cl[p($B_2$+RD)$_{100}$] | solid, brittle | 1.152 | 1.128 | 2.1 |
| Cl[p($B_2$+RD)$_{99}$-stat-pEC$_1$] | solid, soft | 1.161 | 1.129 | 2.9 |
| Cl[p($B_2$+RD)$_{97}$-stat-pEC$_3$] | solid, soft | 1.172 | 1.059 | 10.7 |
| Cl[p($B_2$+RD)$_{93}$-stat-pEC$_7$] | solid, soft | 1.192 | 0.989 | 20.5 |
| Cl[p($B_2$+RD)$_{86}$-stat-pEC$_{14}$] | rubbery | 1.208 | 0.938 | 28.9 |
| Cl[p($B_2$+RD)$_{70}$-stat-pEC$_{30}$] | rubbery | 1.219 | 0.962 | 26.7 |

(continued)

| Identifier | Appearance | $\delta_{Monomer}$ [g×cm$^{-1}$] | $\delta_{Polymer}$ [g×cm$^{-1}$] | Expansion rate [%] |
|---|---|---|---|---|
| Cl[p($B_2$+RD)$_{30}$-stat-pEC$_{70}$] | rubbery, swollen | 1.231 | 1.182 | 4.1 |
| Cl[p($B_2$+RD)$_1$-stat-pEC$_{99}$] | liquid | 1.266 | 1.264 | 0.1 |
| pEC | liquid, highly viscous | 1.311 | 1.321 | -0.8 |
| [p($B_2$+RD)$_{86}$-*stat*-pEC$_{14}$] | rubbery | 1.191 | 1.036 | 15 |

Fig. 4 shows the expansion rate in [%] as a function of the concentration of the cyclic carbonate in the copolymer Cl[p($B_2$+RD)$_{0-100}$-*stat*-pEC$_{0-100}$].

**3.B.4. Cl[p($B_2$+RD)$_i$-*stat*-pPC$_{100-i}$] (i = 100, 99, 97, 93, 86, 70, 30, 1, 0) & [p($B_2$+RD)$_i$-*stat*-pPC$_{100-i}$] (i = 86)**

Table 13:

| Identifier | Appearance | $\delta_{Monomer}$ [g×cm$^{-1}$] | $\delta_{Polymer}$ [g×cm$^{-1}$] | Expansion rate [%] |
|---|---|---|---|---|
| Cl[p($B_2$+RD)$_{100}$] | solid, brittle | 1.152 | 1.128 | 2.1 |
| Cl[p($B_2$+RD)$_{99}$-stat-pPC$_1$] | solid, soft | 1.176 | 1.149 | 2.3 |
| Cl[p($B_2$+RD)$_{97}$-stat-pPC$_3$] | solid, soft | 1.183 | 1.089 | 8.6 |
| Cl[p($B_2$+RD)$_{93}$-stat-pPC$_7$] | solid, soft | 1.19 | 1.003 | 18.6 |
| Cl[p($B_2$+RD)$_{86}$-stat-pPC$_{14}$] | rubbery | 1.201 | 0.944 | 27.2 |
| Cl[p($B_2$+RD)$_{70}$-stat-pPC$_{30}$] | rubbery | 1.209 | 0.936 | 29.2 |
| Cl[p($B_2$+RD)$_{30}$-stat-pPC$_{70}$] | rubbery, swollen | 1.212 | 1.176 | 3.0 |
| Cl[p($B_2$+RD)$_1$-stat-pPC$_{99}$] | liquid | 1.225 | 1.225 | 0 |
| pPC | liquid, highly viscous | 1.229 | 1.235 | -0.5 |
| [p($B_2$+RD)$_{86}$-*stat*-pPC$_{14}$] | rubbery | 1.18 | 1.038 | 13.7 |

Fig. 5 shows the expansion rate in [%] as a function of the concentration of the cyclic carbonate in the copolymer Cl[p($B_2$+RD)$_{0-100}$-*stat*-pPC$_{0-100}$].

**3.B.5. Cl[p($B_3$+RD)$_i$-*stat*-pEC$_{100-i}$] (i = 100, 99, 97, 93, 86, 70, 30, 1, 0)**

Table 14:

| Identifier | Appearance | $\delta_{Monomer}$ [g×cm$^{-1}$] | $\delta_{Polymer}$ [g×cm$^{-1}$] | Expansion rate [%] |
|---|---|---|---|---|
| Cl[p($B_3$+RD)$_{100}$] | solid, brittle | 1.169 | 1.158 | 1.0 |
| Cl[p($B_3$+RD)$_{99}$-stat-pEC$_1$] | solid, soft | 1.171 | 1.146 | 2.1 |
| Cl[p($B_3$+RD)$_{97}$-stat-pEC$_3$] | solid, soft | 1.179 | 1.124 | 4.9 |
| Cl[p($B_3$+RD)$_{93}$-stat-pEC$_7$] | solid, soft | 1.188 | 1.07 | 11 |
| Cl[p($B_3$+RD)$_{86}$-stat-pEC$_{14}$] | rubbery | 1.199 | 1.061 | 13 |
| Cl[p($B_3$+RD)$_{70}$-stat-pEC$_{30}$] | rubbery | 1.205 | 1.072 | 12.4 |
| Cl[p($B_3$+RD)$_{30}$-stat-pEC$_{70}$] | rubbery, swollen | 1.226 | 1.181 | 3.8 |
| Cl[p($B_3$+RD)$_1$-stat-pEC$_{99}$] | liquid | 1.25 | 1.251 | -0.1 |
| pEC | liquid, highly viscous | 1.311 | 1.321 | -0.8 |

Fig. 6 shows the expansion rate in [%] as a function of the concentration of the cyclic carbonate in the copolymer Cl[p($B_3$+RD)$_{0-100}$-*stat*-pEC$_{0-100}$].

**3.B.6. $Cl[p(B_3+RD)_i$-*stat*-$pPC_{100-i}]$ (i = 100, 99, 97, 93, 86, 70, 30, 1, 0)**

Table 15:

| Identifier | Appearance | $\delta_{Monomer}$ [g×cm-1] | $\delta_{Polymer}$ [g×cm-1] | Expansion rate [%] |
|---|---|---|---|---|
| $Cl[p(B_3+RD)_{100}]$ | solid, brittle | 1.169 | 1.158 | 1.0 |
| $Cl[p(B_3+RD)_{99}$-stat-$pPC_1]$ | solid, soft | 1.156 | 1.146 | 0.8 |
| $Cl[p(B_3+RD)_{97}$-stat-$pPC_3]$ | solid, soft | 1.168 | 1.137 | 2.7 |
| $Cl[p(B_3+RD)_{93}$-stat-$pPC_7]$ | solid, soft | 1.177 | 1.105 | 6.6 |
| $Cl[p(B_3+RD)_{86}$-stat-$pPC_{14}]$ | rubbery | 1.189 | 1.055 | 12.8 |
| $Cl[p(B_3+RD)_{70}$-stat-$pPC_{30}]$ | rubbery | 1.199 | 1.095 | 9.5 |
| $Cl[p(B_3+RD)_{30}$-stat-$pPC_{70}]$ | rubbery, swolen | 1.219 | 1.183 | 3.0 |
| $Cl[p(B_3+RD)_1$-stat-$pPC_{99}]$ | liquid | 1.226 | 1.231 | -0.4 |
| pPC | liquid, highly viscous | 1.229 | 1.235 | -0.5 |

Fig. 7 shows the expansion rate in [%] as a function of the concentration of the cyclic carbonate in the copolymer $Cl[p(B_3+RD)_{0-100}$-*stat*-$pPC_{0-100}]$.

**3.B.7. $Cl[p(B_4+RD)_i$-*stat*-$pEC_{100-i}]$ (i = 100, 99, 97, 93, 86, 70, 30, 1, 0)**

Table 16:

| Identifier | Appearance | $\delta_{Monomer}$ [g×cm-1] | $\delta_{Polymer}$ [g×cm-1] | Expansion rate [%] |
|---|---|---|---|---|
| $Cl[p(B_4+RD)_{100}]$ | solid, brittle | 1.207 | 1.204 | 0.2 |
| $Cl[p(B_4+RD)_{99}$-stat-$pEC_1]$ | solid, soft | 1.211 | 1.197 | 1.2 |
| $Cl[p(B_4+RD)_{97}$-stat-$pEC_3]$ | solid, soft | 1.218 | 1.198 | 1.6 |
| $Cl[p(B_4+RD)_{93}$-stat-$pEC_7]$ | rubbery | 1.226 | 1.179 | 4 |
| $Cl[p(B_4+RD)_{86}$-stat-$pEC_{14}]$ | rubbery | 1.235 | 1.135 | 8.8 |
| $Cl[p(B_4+RD)_{70}$-stat-$pEC_{30}]$ | rubbery | 1.242 | 1.129 | 10 |
| $Cl[p(B_4+RD)_{30}$-stat-$pEC_{70}]$ | liquid | 1.247 | 1.179 | 5.8 |
| $Cl[p(B_4+RD)_1$-stat-$pEC_{99}]$ | liquid | 1.253 | 1.254 | -0.1 |
| pEC | liquid, highly viscous | 1.311 | 1.321 | -0.8 |

Fig. 8 shows the expansion rate in [%] as a function of the concentration of the cyclic carbonate in the copolymer $Cl[p(B_4+RD)_{0-100}$-*stat*-$pEC_{0-100}]$.

**3.B.8. $Cl[p(B_4+RD)_i$-*stat*-$pPC_{100-i}]$ (i = 100, 99, 97, 93, 86, 70, 30, 1, 0)**

Table 17:

| Identifier | Appearance | $\delta_{Monomer}$ [g×cm-1] | $\delta_{Polymer}$ [g×cm-1] | Expansion rate [%] |
|---|---|---|---|---|
| $Cl[p(B_4+RD)_{100}]$ | solid, brittle | 1.207 | 1.204 | 0.2 |
| $Cl[p(B_4+RD)_{99}$-stat-$pPC_1]$ | solid, soft | 1.202 | 1.187 | 1.2 |
| $Cl[p(B_4+RD)_{97}$-stat-$pPC_3]$ | solid, soft | 1.206 | 1.183 | 1.9 |
| $Cl[p(B_4+RD)_{93}$-stat-$pPC_7]$ | rubbery | 1.214 | 1.178 | 3.1 |
| $Cl[p(B_4+RD)_{86}$-stat-$pPC_{14}]$ | rubbery | 1.231 | 1.182 | 4.1 |
| $Cl[p(B_4+RD)_{70}$-stat-$pPC_{30}]$ | rubbery | 1.239 | 1.168 | 6 |
| $Cl[p(B_4+RD)_{30}$-stat-$pPC_{70}]$ | liquid | 1.249 | 1.212 | 3 |
| $Cl[p(B_4+RD)_1$-stat-$pPC_{99}]$ | liquid | 1.259 | 1.255 | 0.3 |

(continued)

| Identifier | Appearance | $\delta_{Monomer}$ [g$\times$cm$^{-1}$] | $\delta_{Polymer}$ [g$\times$cm$^{-1}$] | Expansion rate [%] |
|---|---|---|---|---|
| pPC | liquid, highly viscous | 1.229 | 1.235 | -0.5 |

Fig. 9 shows the expansion rate in [%] as a function of the concentration of the cyclic carbonate in the copolymer Cl[p($B_4$+RD)$_{0-100}$-***stat***-pPC$_{0-100}$].

3.C **Conclusion**

Benzoxazines and cyclic carbonates can be copolymerized according to ring-opening mechanisms. During this curing step, the formulation exhibits volumetric expansion of up to more than 30 vol.-%. Notably, the corresponding homopolymers show volumetric expansion to low extent only (in the case of poly(benzoxazine)s) or no volumetric expansion at all (in the case of poly(cyclic carbonate)s). This behavior of formulations containing benzoxazines and cyclic carbonates is in contrast to numerous other monomer formulations that show shrinkage during the curing step. The extent of volumetric expansion can be tailored in the range from 0 to more than 30 vol.-% by careful choice of the amount and types of benzoxazines and cyclic carbonates. Correspondingly, also the mechanic properties of the corresponding copolymers can be varied from highly brittle to rubber-like types.

The formulation containing the benzoxazines and cyclic carbonates is liquid to semi-solid; its viscosity can be adjusted by the addition of reactive diluents, for example benzoxazine-based reactive diluents. Hence, solvent-free mixtures can be maintained. As such, these formulations can be used as coatings and adhesives that expand during the curing reaction, yielding void-free and crack-free coatings that do not delaminate from the substrate they were adhered to. If the benzoxazines contain additional functional groups such as olefinic units that enable crosslinking by reactions such as the thiol-ene click reaction, the formulation can be pre-cured such that the crosslinking is performed prior to the ring-opening copolymerization. One possible technique for this strategy is the addition of radical photoinitiators and the application of UV irradiation such that crosslinking can be performed at room temperature, while the temperature-inducible ring-opening copolymerization does not yet start. By this strategy, solid specimens with tailor-made geometry can be produced, which can be inserted into cavities, in which they show geometric alignment (e.g., complete filling of the cavity) upon temperature stimuli.

## 4. Application Examples

### 4.1. Application Example 1

A precured specimen of the composition Cl[p($B_2$+RD)$_{93}$-stat-pEC$_7$] and dimensions in the centi- and decimeter range, e.g. 11.8 $\times$ 3.9 $\times$ 0.95 cm, can be used as support and fixation material for windings of electrical machines. This specimen is inserted into a cavity with slightly larger dimensions than those of the specimen, e.g. 12 $\times$ 4 $\times$ 1 cm. Subsequently, heat is applied, and the specimen expands volumetrically due to the ring-opening polymerization. Due to the volumetric expansion, precise geometric alignment of the specimen to the walls of the cavity occurs, which renders excellent stability and, additionally, insulating properties, as no cracks or voids are formed between the specimen and the walls of the cavity.

### 4.2. Application Example 2

A pre-cured specimen of the composition **Cl[p($B_2$+RD)$_{86}$-*stat*-pPC$_{14}$]** and dimensions in the milli-, centi-, or meter range in the form of, e.g., finished bars, sticks, cylinders or spacers, can be used as support and fixation material in high voltage winding insulation barrier systems of electrical equipment, which is liquid or gas insulated. This specimen is inserted into insulation barrier systems with other conventional insulation material. Subsequently, heat is applied during a dry-out process, and the specimen expands volumetrically due to the ring-opening polymerization. Due to the volumetric expansion, precise geometric alignment of the specimen to the surrounding material occurs and compensates the shrinkage and assembling tolerances of those materials, which renders excellent stability and, additionally, insulating properties and form fit support of, e.g., windings or laminated electromagnetic materials.

## Claims

**1.** An expandable, polymerizable composition comprising at least one benzoxazine and at least one cyclic carbonate,

**characterized in that** the benzoxazine units comprised in the composition are benzoxazine units which are crosslinkable with each other.

2. The expandable, polymerizable composition according to claim 1, **characterized in that** the benzoxazines are crosslinkable by thiol-ene crosslinking.

3. The expandable, polymerizable composition according to claim 1 or 2, wherein the crosslinkable benzoxazine is a compound of general Formula (II):

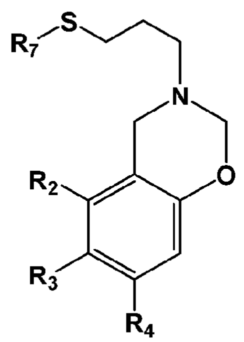

Formula (II)

wherein $R_2$, $R_3$, and $R_4$ are each independently H, $CH_3$, $C_2$-$C_{15}$ straight, branched or cyclic alkyl optionally substituted with halogens, alkenyl or alkynyl groups, alkaryl groups, heteroalkyl groups, heteroaryl groups, hydroxy groups, disulfides, sulfonates, ether groups, thioether groups, ester groups, carboxylic acid groups, amine groups, amide groups, azides, or benzoxazines, and $R_2$ can be linked with $R_3$ to form a cyclic substituent on the benzene ring or $R_3$ can be linked with $R_4$ to form a cyclic substituent on the benzene ring, with the proviso that $R_2$, $R_3$, and/or $R_4$ comprises at least one unsaturated bond; and
$R_7$ is $C_2$-$C_{15}$ straight, branched or cyclic alkyl optionally substituted with halogens, alkaryl groups, heteroalkyl groups, heteroaryl groups, hydroxy groups, disulfides, sulfonates, ether groups, thioether groups, ester groups, carboxylic acid groups, amine groups, amide groups and azides, with the proviso that $R_7$ comprises at least one thiol group.

4. The expandable, polymerizable composition according to any one of claims 1 to 3, **characterized in that** the cyclic carbonate is a compound of general Formula (III):

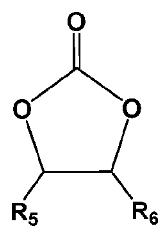

Formula (III)

wherein $R_5$ and $R_6$ are each independently H, $CH_3$, $C_2$-$C_{15}$ straight, branched or cyclic alkyl optionally substituted with halogens, alkenyl, heteroalkyl groups and hydroxy groups.

5. The expandable, polymerizable composition according to claim 4, **characterized in that** the cyclic carbonate is ethylene carbonate or propylene carbonate.

6. The expandable, polymerizable composition according to any one of claims 1 to 5, **characterized in that** the benzoxazine is derived from a dihydroxybenzene or a bisphenol, preferably selected from the group consisting of

hydroquinone, Bisphenol A, Bisphenol F, Bisphenol S, Bisphenol M, Bisphenol Z, Bisphenol AP.

7. The expandable, polymerizable composition according any one of claims 1 to 6, **characterized in that** the ratio of benzoxazine equivalents to cyclic carbonate equivalents in the composition is from 99:1 to 1:99, preferably from 99:1 to 30:70.

8. The expandable, polymerizable composition according any one of claims 1 to 7, **characterized in that** the composition additionally comprises at least one reactive diluent.

9. The expandable, polymerizable composition according to claim 8, **characterized in that** the reactive diluent is present at between 20% to 60% by weight of the benzoxazine.

10. The expandable, polymerizable composition according to claim 8 or 9, **characterized in that** the reactive diluent is selected from the group consisting of 3-allyl-3,4-dihydro-2H-benzo[e][1,3]oxazine, 3-allyl-5-methyl-3,4-dihydro-2H-benzo [e] [1,3] oxazine, 3-allyl-6-octyl-3,4-dihydro-2H-benzo[e][1,3]oxazine and 3-allyl-6-nonyl-3,4-dihydro-2H-benzo[e][1,3]oxazine.

11. A poly(benzoxazine)-co-poly(cyclic carbonate) polymerization product of the expandable, polymerizable composition according to any one of claims 1 to 10.

12. The polymerization product according to claim 11, comprising a poly(benzoxazine)-co-poly(cyclic carbonate) of the general formula (V):

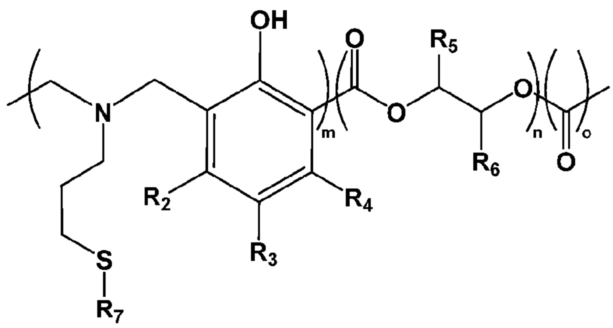

Formula (V)

wherein

$R_2$, $R_3$, and $R_4$ are each independently H, $CH_3$, $C_2$-$C_{15}$ straight, branched or cyclic alkyl optionally substituted with halogens, alkenyl or alkynyl groups, alkaryl groups, heteroalkyl groups, heteroaryl groups, hydroxy groups, disulfides, sulfonates, ether groups, thioether groups, ester groups, carboxylic acid groups, amine groups, amide groups, azides, or benzoxazine, and $R_2$ can be linked with $R_3$ to form a cyclic substituent on the benzene ring or $R_3$ can be linked with $R_4$ to form a cyclic substituent on the benzene ring, with the proviso that $R_2$, $R_3$, and/or $R_4$ comprises at least one unsaturated bond;
$R_7$ is $C_2$-$C_{15}$ straight, branched or cyclic alkyl optionally substituted with halogens, alkaryl groups, heteroalkyl groups, heteroaryl groups, hydroxy groups, disulfides, sulfonates, ether groups, thioether groups, ester groups, carboxylic acid groups, amine groups, amide groups and azides, with the proviso that $R_7$ comprises at least one thiol group;
$R_5$ and $R_6$ are each independently H, $CH_3$, $C_2$-$C_{15}$ straight, branched or cyclic alkyl optionally substituted with halogens, alkenyl, heteroalkyl groups and hydroxy groups;
m is an integer between 10 and 10'000;
n is an integer between 10 and 6'700;
o is an integer between 0 and 1000.

13. A sealant, adhesive, coating, binding agent or dental filling comprising an expandable polymerizable composition according to any one of claims 1 to 10.

**14.** Use of an expandable, polymerizable composition according to any one of claims 1 to 10 as/in sealants, adhesives, coatings, binding agents or dental fillings.

**15.** Use of an expandable, polymerizable composition comprising at least one benzoxazine and at least one cyclic carbonate in the manufacture of a poly(benzoxazine)-co-poly(cyclic carbonate), wherein the expandable, polymerizable composition shows volumetric expansion during copolymerization upon a heat stimulus.

**16.** The use of an expandable, polymerizable composition according to claim 15, **characterized in that** the benzoxazine is a compound of general Formula (I):

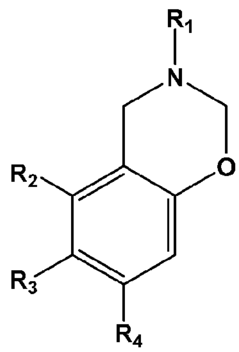

**Formula (I)**

wherein $R_1$, $R_2$, $R_3$, and $R_4$ are each independently H, $CH_3$, $C_2$-$C_{15}$ straight, branched or cyclic alkyl optionally substituted with halogens, alkenyl or alkynyl groups, alkaryl groups, heteroalkyl groups, heteroaryl groups, hydroxy groups, disulfides, sulfonates, ether groups, thioether groups, ester groups, carboxylic acid groups, amine groups, amide groups, azides, or benzoxazines, and $R_2$ can be linked with $R_3$ to form a cyclic substituent on the benzene ring or $R_3$ can be linked with $R_4$ to form a cyclic substituent on the benzene ring.

**17.** The use of an expandable, polymerizable composition according to claim 15 or 16, **characterized in that** the cyclic carbonate is a compound of general Formula (III):

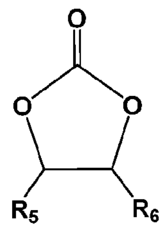

**Formula (III)**

wherein $R_5$ and $R_6$ are each independently H, $CH_3$, $C_2$-$C_{15}$ straight, branched or cyclic alkyl optionally substituted with halogens, alkenyl, heteroalkyl groups and hydroxy groups.

**18.** The use of an expandable, polymerizable composition according to claim 17, **characterized in that** the cyclic carbonate is ethylene carbonate or propylene carbonate.

**19.** The use of an expandable, polymerizable composition according to any one of claims 15 to 18, **characterized in that** the benzoxazine is derived from a dihydroxybenzene or a bisphenol, preferably selected from the group consisting of hydroquinone, Bisphenol A, Bisphenol F, Bisphenol S, Bisphenol M, Bisphenol Z, Bisphenol AP.

**20.** The use of an expandable, polymerizable composition according any one of claims 15 to 19, **characterized in that**

the ratio of benzoxazine equivalents to cyclic carbonate equivalents in the composition is from 99:1 to 1:99, preferably from 99:1 to 30:70.

**21.** The use of an expandable, polymerizable composition according any one of claims 15 to 20, **characterized in that** the composition additionally comprises at least one reactive diluent.

**22.** The use of an expandable, polymerizable composition according to claim 21, **characterized in that** the reactive diluent is present at between 20% to 60% by weight of the benzoxazine.

**23.** The use of an expandable, polymerizable composition according to claim 21 or 22, **characterized in that** the reactive diluent is selected from the group consisting of 3-allyl-3,4-dihydro-2H-benzo [e] [1,3] oxazine, 3-allyl-5-methyl-3,4-dihydro-2H-benzo [e] [1,3] oxazine, 3-allyl-6-octyl-3,4-dihydro-2H-benzo[e][1,3]oxazine and 3-allyl-6-nonyl-3,4-dihydro-2H-benzo[e] [1,3]oxazine.

**24.** The use of an expandable, polymerizable composition according to any one of claims 15 to 23 as/in sealants, adhesives, coatings, binding agents or dental fillings.

**25.** A poly(benzoxazine)-co-poly(cyclic carbonate) polymerization product obtained by copolymerizing an expandable, polymerizable composition as defined in any one of claims 15 to 23 at a temperature sufficient to initiate copolymerization.

**26.** The poly(benzoxazine)-co-poly(cyclic carbonate) polymerization product according to claim 25, comprising a poly(benzoxazine)-co-poly(cyclic carbonate) of the general formula (IV):

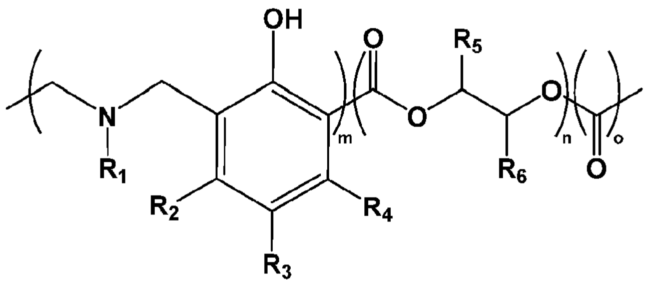

**Formula (IV)**

wherein

$R_1$, $R_2$, $R_3$, and $R_4$ are each independently H, $CH_3$, $C_2$-$C_{15}$ straight, branched or cyclic alkyl optionally substituted with halogens, alkenyl or alkynyl groups, alkaryl groups, heteroalkyl groups, heteroaryl groups, hydroxy groups, disulfides, sulfonates, ether groups, thioether groups, ester groups, carboxylic acid groups, amine groups, amide groups, azides, or benzoxazine, and $R_2$ can be linked with $R_3$ to form a cyclic substituent on the benzene ring or $R_3$ can be linked with $R_4$ to form a cyclic substituent on the benzene ring;
$R_5$ and $R_6$ are each independently H, $CH_3$, $C_2$-$C_{15}$ straight, branched or cyclic alkyl optionally substituted with halogens, alkenyl, heteroalkyl groups and hydroxy groups;
m is an integer between 10 and 10'000;
n is an integer between 10 and 6'700;
o is an integer between 0 and 1'000.

**27.** A process for manufacturing a poly(benzoxazine)-co-poly(cyclic carbonate) polymerization product, comprising the step of heating an expandable, polymerizable composition according to any one of claims 1 to 10 or an expandable, polymerizable composition as defined in any one of claims 15 to 23 to a temperature sufficient to initiate copolymerization.

**28.** The process according to claim 27, comprising the steps of:

• providing a polymerizable composition according to any one of claims 1 to 10, comprising crosslinkable benzoxazines and cyclic carbonates,
• precuring the polymerizable composition by crosslinking the crosslinkable benzoxazines, and
• heating the precured polymerizable composition to a temperature sufficient to initiate copolymerization of the crosslinked benzoxazines and cyclic carbonates.

**29.** A precured, expandable, polymerizable composition obtained by crosslinking crosslinkable benzoxazines comprised in an expandable polymerizable composition according to any one of claims 1 to 10.

**30.** Use of a precured, expandable, polymerizable composition according to claim 29, as an expandable filling element for filling spaces in devices, wherein the precured, expandable composition has a pre-defined form, and wherein the precured, expandable composition shows volumetric expansion during copolymerization upon a heat stimulus.

**31.** Use of a precured, expandable, polymerizable composition according to claim 30 as a support and fixation element for windings of electrical machines or as a winding insulation barrier for electrical equipment.

## Patentansprüche

**1.** Expandierbare, polymerisierbare Zusammensetzung, die mindestens ein Benzoxazin und mindestens ein cyclisches Carbonat enthält, **dadurch gekennzeichnet, daß** die in der Zusammensetzung enthaltenen Benzoxazin-Einheiten Benzoxazin-Einheiten sind, die miteinander vernetzbar sind.

**2.** Expandierbare, polymerisierbare Zusammensetzung nach Anspruch 1, **dadurch gekennzeichnet, daß** die Benzoxazine durch Thiol-En-Vernetzung vernetzbar sind.

**3.** Expandierbare, polymerisierbare Zusammensetzung nach Anspruch 1 oder 2, wobei das vernetzbare Benzoxazin eine Verbindung der allgemeinen Formel (II) ist:

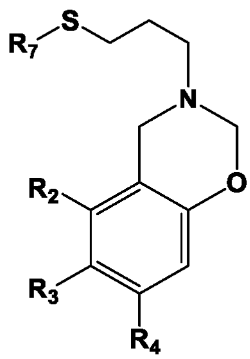

Formel (II)

worin $R_2$, $R_3$, und $R_4$ jeweils unabhängig voneinander H, $CH_3$, $C_2$-$C_{15}$ geradkettiges, verzweigtes oder cyclisches Alkyl, gegebenenfalls substituiert mit Halogenen, Alkenyl- oder Alkinylgruppen, Alkarylgruppen, Heteroalkylgruppen, Heteroarylgruppen, Hydroxygruppen, Disulfiden, Sulfonaten, Ethergruppen, Thioethergruppen, Estergruppen, Carbonsäuregruppen, Amingruppen, Amidgruppen, Aziden oder Benzoxazinen sind, und $R_2$ kann mit $R_3$ verbunden sein, um einen cyclischen Substituenten am Benzolring zu bilden, oder $R_3$ kann mit $R_4$ verbunden sein, um einen cyclischen Substituenten am Benzolring zu bilden, mit der Maßgabe, dass $R_2$, $R_3$ und/oder $R_4$ mindestens eine ungesättigte Bindung umfasst; und
$R_7$ ist $C_2$-$C_{15}$geradkettiges, verzweigtes oder cyclisches Alkyl, das gegebenenfalls mit Halogenen, Alkarylgruppen, Heteroalkylgruppen, Heteroarylgruppen, Hydroxygruppen, Disulfiden, Sulfonaten, Ethergruppen, Thioethergruppen, Estergruppen, Carbonsäuregruppen, Amingruppen, Amidgruppen und Aziden substituiert ist, mit der Maßgabe, dass $R_7$ mindestens eine Thiolgruppe umfasst.

**4.** Expandierbare, polymerisierbare Zusammensetzung nach einem der Ansprüche 1 bis 3, **dadurch gekennzeichnet, dass** das cyclische Carbonat eine Verbindung der allgemeinen Formel (III) ist:

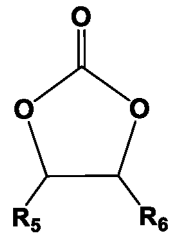

Formel (III)

worin $R_5$ und $R_6$ jeweils unabhängig voneinander H, $CH_3$, $C_2$-$C_{15}$ geradkettiges, verzweigtes oder cyclisches Alkyl, gegebenenfalls substituiert mit Halogenen, Alkenyl-, Heteroalkyl- und Hydroxygruppen sind.

5. Expandierbare, polymerisierbare Zusammensetzung nach Anspruch 4, **dadurch gekennzeichnet, daß** das cyclische Carbonat Ethylencarbonat oder Propylencarbonat ist.

6. Expandierbare, polymerisierbare Zusammensetzung nach einem der Ansprüche 1 bis 5, **dadurch gekennzeichnet, daß** das Benzoxazin von einem Dihydroxybenzol oder einem Bisphenol abgeleitet ist, vorzugsweise ausgewählt aus der Gruppe bestehend aus Hydrochinon, Bisphenol A, Bisphenol F, Bisphenol S, Bisphenol M, Bisphenol Z, Bisphenol AP.

7. Expandierbare, polymerisierbare Zusammensetzung nach einem der Ansprüche 1 bis 6, **dadurch gekennzeichnet, daß** das Verhältnis von Benzoxazin-Äquivalenten zu cyclischen Carbonat-Äquivalenten in der Zusammensetzung 99:1 bis 1:99, vorzugsweise 99:1 bis 30:70 beträgt.

8. Expandierbare, polymerisierbare Zusammensetzung nach einem der Ansprüche 1 bis 7, **dadurch gekennzeichnet, dass** die Zusammensetzung zusätzlich mindestens einen Reaktivverdünner enthält.

9. Expandierbare, polymerisierbare Zusammensetzung nach Anspruch 8, **dadurch gekennzeichnet, dass** der Reaktivverdünner in einer Menge von 20 bis 60 Gew.-% des Benzoxazins vorhanden ist.

10. Expandierbare, polymerisierbare Zusammensetzung nach Anspruch 8 oder 9, **dadurch gekennzeichnet, dass** der Reaktivverdünner ausgewählt ist aus der Gruppe bestehend aus 3-Allyl-3,4-dihydro-2H-benzo[e][1,3]oxazin, 3-allyl-5-methyl-3,4-dihydro-2H-benzo[e][1,3]oxazin, 3-allyl-6-octyl-3,4-dihydro-2H-benzo[e] [1,3]oxazin und 3-allyl-6-nonyl-3,4-dihydro-2H-benzo[e] [1,3]oxazin.

11. Poly(benzoxazin)-co-poly(cyclisches Carbonat)-Polymerisationsprodukt der expandierbaren, polymerisierbaren Zusammensetzung nach einem der Ansprüche 1 bis 10.

12. Polymerisationsprodukt nach Anspruch 11, umfassend ein Poly(benzoxazin)-co-poly(cyclisches Carbonat) der allgemeinen Formel (V):

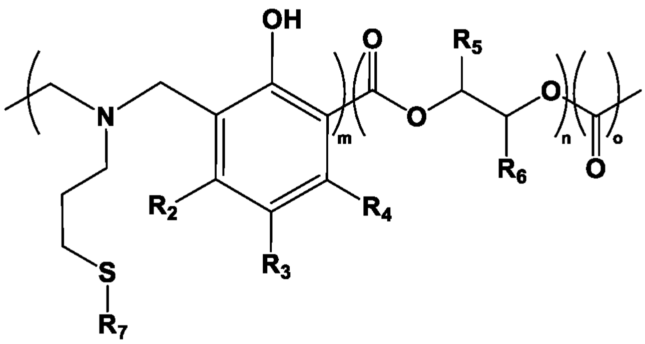

Formel (V)

worin

$R_2$, $R_3$, und $R_4$ jeweils unabhängig voneinander H, $CH_3$, $C_2$-$C_{15}$ geradkettiges, verzweigtes oder cyclisches Alkyl, gegebenenfalls substituiert mit Halogenen, Alkenyl- oder Alkinylgruppen, Alkarylgruppen, Heteroalkylgruppen, Heteroarylgruppen, Hydroxygruppen, Disulfiden, Sulfonaten, Ethergruppen, Thioethergruppen, Estergruppen, Carbonsäuregruppen, Amingruppen, Amidgruppen, Aziden oder Benzoxazin sind, und $R_2$ kann mit $R_3$ verbunden sein, um einen cyclischen Substituenten am Benzolring zu bilden, oder $R_3$ kann mit $R_4$ verbunden sein, um einen cyclischen Substituenten am Benzolring zu bilden, mit der Maßgabe, dass $R_2$, $R_3$ und/oder $R_4$ mindestens eine ungesättigte Bindung umfasst;
$R_7$ ist $C_2$-$C_{15}$geradkettiges, verzweigtes oder cyclisches Alkyl ist, das gegebenenfalls mit Halogenen, Alkarylgruppen, Heteroalkylgruppen, Heteroarylgruppen, Hydroxygruppen, Disulfiden, Sulfonaten, Ethergruppen, Thioethergruppen, Estergruppen, Carbonsäuregruppen, Amingruppen, Amidgruppen und Aziden substituiert ist, mit der Maßgabe, dass $R_7$ mindestens eine Thiolgruppe umfasst;
$R_5$ und $R_6$ jeweils unabhängig voneinander H, $CH_3$, $C_2$-$C_{15}$ geradkettiges, verzweigtes oder zyklisches Alkyl, gegebenenfalls substituiert mit Halogenen, Alkenyl-, Heteroalkylgruppen und Hydroxygruppen, sind;
m eine ganze Zahl zwischen 10 und 10'000 ist;
n eine ganze Zahl zwischen 10 und 6'700 ist;
o eine ganze Zahl zwischen 0 und 1000 ist.

**13.** Dichtungsmittel, Klebstoff, Beschichtung, Bindemittel oder Zahnfüllung, umfassend eine expandierbare polymerisierbare Zusammensetzung nach einem der Ansprüche 1 bis 10.

**14.** Verwendung einer expandierbaren, polymerisierbaren Zusammensetzung nach einem der Ansprüche 1 bis 10 als/für Dichtungsmittel, Klebstoffe, Beschichtungen, Bindemittel oder Zahnfüllungen.

**15.** Verwendung einer expandierbaren, polymerisierbaren Zusammensetzung, die mindestens ein Benzoxazin und mindestens ein zyklisches Carbonat enthält, bei der Herstellung eines Poly(benzoxazin)-Co-Poly(zyklisches Carbonat), wobei die expandierbare, polymerisierbare Zusammensetzung während der Copolymerisation auf einen Wärmereiz hin eine volumetrische Expansion zeigt.

**16.** Verwendung einer expandierbaren, polymerisierbaren Zusammensetzung nach Anspruch 15, **dadurch gekennzeichnet, dass** das Benzoxazin eine Verbindung der allgemeinen Formel (I) ist:

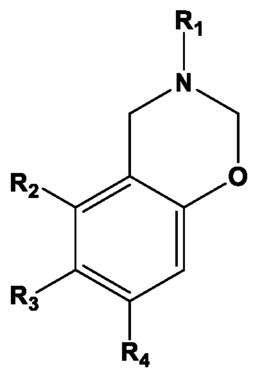

Formel (I)

worin $R_1$, $R_2$, $R_3$, und $R_4$ jeweils unabhängig voneinander H, $CH_3$, $C_2$-$C_{15}$ geradkettiges, verzweigtes oder zyklisches Alkyl, gegebenenfalls substituiert mit Halogenen, Alkenyl- oder Alkinylgruppen, Alkarylgruppen, Heteroalkylgruppen, Heteroarylgruppen, Hydroxygruppen, Disulfiden, Sulfonaten, Ethergruppen, Thioethergruppen, Estergruppen, Carbonsäuregruppen, Amingruppen, Amidgruppen, Aziden oder Benzoxazinen sind, und $R_2$ mit $R_3$ verbunden sein kann, um einen cyclischen Substituenten am Benzolring zu bilden, oder $R_3$ mit $R_4$ verbunden sein kann, um einen cyclischen Substituenten am Benzolring zu bilden.

**17.** Verwendung einer expandierbaren, polymerisierbaren Zusammensetzung nach Anspruch 15 oder 16, **dadurch gekennzeichnet, dass** das cyclische Carbonat eine Verbindung der allgemeinen Formel (III) ist:

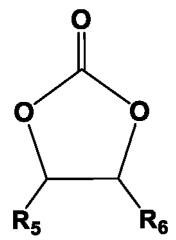

Formel (III)

worin $R_5$ und $R_6$ jeweils unabhängig voneinander H, $CH_3$, $C_2$-$C_{15}$ geradkettiges, verzweigtes oder cyclisches Alkyl, gegebenenfalls substituiert mit Halogenen, Alkenyl-, Heteroalkyl- und Hydroxygruppen sind.

**18.** Verwendung einer expandierbaren, polymerisierbaren Zusammensetzung nach Anspruch 17, **dadurch gekennzeichnet, daß** das cyclische Carbonat Ethylencarbonat oder Propylencarbonat ist.

**19.** Verwendung einer expandierbaren, polymerisierbaren Zusammensetzung nach einem der Ansprüche 15 bis 18, **dadurch gekennzeichnet, daß** das Benzoxazin von einem Dihydroxybenzol oder einem Bisphenol abgeleitet ist, vorzugsweise ausgewählt aus der Gruppe bestehend aus Hydrochinon, Bisphenol A, Bisphenol F, Bisphenol S, Bisphenol M, Bisphenol Z, Bisphenol AP.

**20.** Verwendung einer expandierbaren, polymerisierbaren Zusammensetzung nach einem der Ansprüche 15 bis 19, **dadurch gekennzeichnet, daß** das Verhältnis von Benzoxazin-Äquivalenten zu cyclischen Carbonat-Äquivalenten in der Zusammensetzung 99:1 bis 1:99, vorzugsweise 99:1 bis 30:70 beträgt.

**21.** Verwendung einer expandierbaren, polymerisierbaren Zusammensetzung nach einem der Ansprüche 15 bis 20, **dadurch gekennzeichnet, dass** die Zusammensetzung zusätzlich mindestens einen Reaktivverdünner enthält.

**22.** Verwendung einer expandierbaren, polymerisierbaren Zusammensetzung nach Anspruch 21, **dadurch gekennzeichnet, dass** der Reaktivverdünner zu 20 bis 60 Gew.-% des Benzoxazins vorhanden ist.

**23.** Verwendung einer expandierbaren, polymerisierbaren Zusammensetzung nach Anspruch 21 oder 22, **dadurch gekennzeichnet, dass** der Reaktivverdünner ausgewählt ist aus der Gruppe bestehend aus 3-Allyl-3, 4-dihydro-2H-benzo[e][1,3]oxazin, 3-allyl-5-methyl-3,4-dihydro-2H-benzo[e][1,3]oxazin, 3-allyl-6-octyl-3,4-dihydro-2H-benzo[e][1,3]oxazin und 3-allyl-6-nonyl-3,4-dihydro-2H-benzo [e] [1,3]oxazine.

**24.** Verwendung einer expandierbaren, polymerisierbaren Zusammensetzung nach einem der Ansprüche 15 bis 23 als/für Dichtungsmittel, Klebstoffe, Beschichtungen, Bindemittel oder Zahnfüllungen.

**25.** Poly(benzoxazin)-Co-Poly(cyclisches Carbonat)-Polymerisationsprodukt, erhalten durch CoPolymerisation einer expandierbaren, polymerisierbaren Zusammensetzung nach einem der Ansprüche 15 bis 23 bei einer Temperatur, die ausreicht, um die Copolymerisation einzuleiten.

**26.** Poly(benzoxazin)-co-poly(cyclisches Carbonat)-Polymerisationsprodukt nach Anspruch 25, umfassend ein Poly(benzoxazin)-co-poly(cyclisches Carbonat) der allgemeinen Formel (IV):

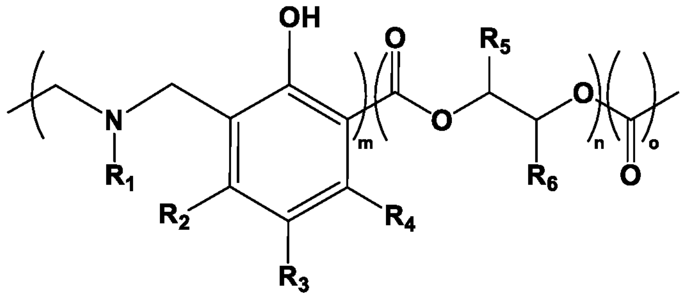

Formel (IV)

worin

$R_1$, $R_2$, $R_3$ und $R_4$ jeweils unabhängig voneinander H, $CH_3$, $C_2$-$C_{15}$ geradkettiges, verzweigtes oder cyclisches Alkyl, gegebenenfalls substituiert mit Halogenen, Alkenyl- oder Alkinylgruppen, Alkarylgruppen, Heteroalkylgruppen, Heteroarylgruppen, Hydroxygruppen, Disulfiden, Sulfonaten, Ethergruppen, Thioethergruppen, Estergruppen, Carbonsäuregruppen, Amingruppen, Amidgruppen, Aziden oder Benzoxazin sind, und $R_2$ kann mit $R_3$ verbunden sein, um einen cyclischen Substituenten am Benzolring zu bilden, oder $R_3$ kann mit $R_4$ verbunden sein, um einen cyclischen Substituenten am Benzolring zu bilden;
$R_5$ und $R_6$ jeweils unabhängig voneinander H, $CH_3$, $C_2$-$C_{15}$ geradkettiges, verzweigtes oder cyclisches Alkyl, gegebenenfalls substituiert mit Halogenen, Alkenyl-, Heteroalkylgruppen und Hydroxygruppen sind
m eine ganze Zahl zwischen 10 und 10'000 ist;
n eine ganze Zahl zwischen 10 und 6'700 ist;
o eine ganze Zahl zwischen 0 und 1'000 ist.

**27.** Verfahren zur Herstellung eines Poly(benzoxazin)-Co-Poly(cyclisches Carbonat)-Polymerisationsprodukts, umfassend den Schritt des Erhitzens einer expandierbaren, polymerisierbaren Zusammensetzung gemäß einem der Ansprüche 1 bis 10 oder einer expandierbaren, polymerisierbaren Zusammensetzung gemäß einem der Ansprüche 15 bis 23 auf eine Temperatur, die ausreicht, um die Copolymerisation einzuleiten.

**28.** Verfahren nach Anspruch 27, umfassend die Schritte:

- Bereitstellen einer polymerisierbaren Zusammensetzung nach einem der Ansprüche 1 bis 10, umfassend vernetzbare Benzoxazine und cyclische Carbonate,
- Vorhärten der polymerisierbaren Zusammensetzung durch Vernetzen der vernetzbaren Benzoxazine, und
- Erhitzen der vorgehärteten polymerisierbaren Zusammensetzung auf eine Temperatur, die ausreicht, um die Copolymerisation der vernetzten Benzoxazine und der cyclischen Carbonate einzuleiten.

**29.** Vorgehärtete, expandierbare, polymerisierbare Zusammensetzung, erhalten durch Vernetzen von vernetzbaren Benzoxazinen, die in einer expandierbaren polymerisierbaren Zusammensetzung nach einem der Ansprüche 1 bis 10 enthalten sind.

**30.** Verwendung einer vorgehärteten, expandierbaren, polymerisierbaren Zusammensetzung nach Anspruch 29 als expandierbares Füllelement zum Füllen von Zwischenräumen in Vorrichtungen, wobei die vorgehärtete, expandierbare Zusammensetzung eine vordefinierte Form aufweist und wobei die vorgehärtete, expandierbare Zusammensetzung während der Copolymerisation auf einen Wärmereiz hin eine volumetrische Expansion zeigt.

**31.** Verwendung einer vorgehärteten, expandierbaren, polymerisierbaren Zusammensetzung nach Anspruch 30 als Stütz- und Fixierelement für Wicklungen elektrischer Maschinen oder als Wicklungsisolationssperre für elektrische Geräte.

## Revendications

**1.** Composition polymérisable et expansible comprenant au moins une benzoxazine et au moins un carbonate cyclique, **caractérisée en ce que** les unités benzoxazine comprises dans la composition sont des unités benzoxazine réticulables entre elles.

2. Composition polymérisable et expansible selon la revendication 1, **caractérisée en ce que** les benzoxazines sont réticulables par une réticulation thiol-ène.

3. Composition polymérisable et expansible selon la revendication 1 ou 2, dans laquelle la benzoxazine réticulable est un composé de la formule générale (**II**) :

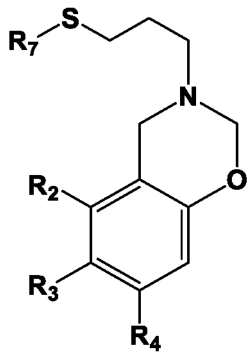

Formule (II)

dans laquelle $R_2$, $R_3$, et $R_4$ sont chacun indépendamment H, $CH_3$, $C_2$-$C_{15}$ alkyle linéaire, ramifié ou cyclique éventuellement substitué par des halogènes, groupes alcényle ou alcynyle, groupes alcaryle, groupes hétéroalkyle, groupes hétéroaryle, groupes hydroxy, disulfures, sulfonates, groupes éther, groupes thioéther, groupes ester, groupes d'acides carboxyliques, groupes d'amines, groupes d'amides, azides ou benzoxazines, et $R_2$ peut être lié à $R_3$ pour former un substituant cyclique sur le cycle benzénique ou $R_3$ peut être lié à $R_4$ pour former un substituant cyclique sur le cycle benzénique, à condition que $R_2$, $R_3$, et/ou $R_4$ comprenne au moins une liaison insaturée ; et

$R_7$ est $C_2$-$C_{15}$ un alkyle linéaire, ramifié ou cyclique éventuellement substitué par des halogènes, des groupes alcaryle, des groupes hétéroalkyles, des groupes hétéroaryles, des groupes hydroxy, des disulfures, des sulfonates, des groupes éther, des groupes thioéther, des groupes ester, des groupes acide carboxylique, des groupes amine, des groupes amide et des azides, à condition que $R_7$ comprenne au moins un groupe thiol.

4. Composition polymérisable et expansible selon l'une quelconque des revendications 1 à 3, **caractérisée en ce que** le carbonate cyclique est un composé de la formule générale (**III**) :

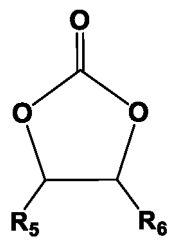

Formule (III)

dans laquelle $R_5$ et $R_6$ sont chacun indépendamment H, $CH_3$, $C_2$-$C_{15}$ alkyle linéaire, ramifié ou cyclique éventuellement substitué par des halogènes, des groupes alcényles, hétéroalkyles et des groupes hydroxy.

5. Composition polymérisable et expansible selon la revendication 4, **caractérisée en ce que** le carbonate cyclique est le carbonate d'éthylène ou le carbonate de propylène.

6. Composition polymérisable et expansible selon l'une quelconque des revendications 1 à 5, **caractérisée en ce que** la benzoxazine est dérivée d'un dihydroxybenzène ou d'un bisphénol, de préférence choisi dans le groupe constitué par l'hydroquinone, le Bisphénol A, le Bisphénol F, le Bisphénol S, le Bisphénol M, le Bisphénol Z, le Bisphénol AP.

**7.** Composition polymérisable et expansible selon l'une quelconque des revendications 1 à 6, **caractérisée en ce que** le rapport entre les équivalents de benzoxazine et les équivalents de carbonate cyclique dans la composition est compris entre 99:1 et 1:99, de préférence entre 99:1 et 30:70.

**8.** Composition polymérisable et expansible selon l'une quelconque des revendications 1 à 7, **caractérisée en ce que** la composition comprend en outre au moins un diluant réactif.

**9.** Composition polymérisable et expansible selon la revendication 8, **caractérisée en ce que** le diluant réactif est présent entre 20 % et 60 % en poids de la benzoxazine.

**10.** Composition polymérisable et expansible selon la revendication 8 ou 9,
**caractérisée en ce que** le diluant réactif est choisi dans le groupe constitué de 3-allyl-3,4-dihydro-2H-benzo[e][1,3]oxazine, 3-allyl-5-methyl-3,4-dihydro-2H-benzo[e][1,3]oxazine, 3-allyl-6-octyl-3,4-dihydro-2H-benzo[e][1,3]oxazine and 3-allyl-6-nonyl-3,4-dihydro-2H-benzo [e] [1,3]oxazine.

**11.** Produit de polymérisation poly(benzoxazine)-co-poly(carbonate cyclique) de la composition polymérisable et expansible selon l'une quelconque des revendications 1 à 10.

**12.** Produit de polymérisation selon la revendication 11, comprenant un poly(benzoxazine)-co-poly(carbonate cyclique) de formule générale (**V**) :

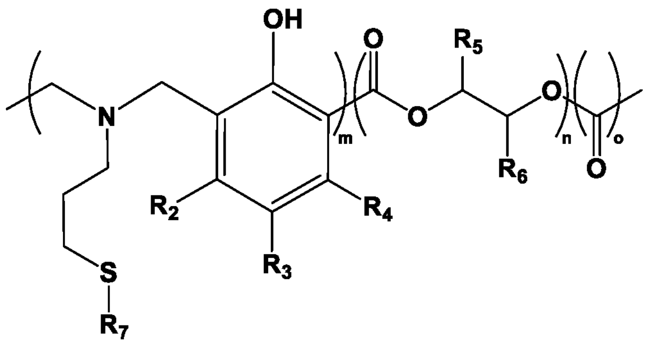

Formule (V)

dans laquelle

$R_2$, $R_3$, et $R_4$ sont chacun indépendamment H, $CH_3$, $C_2$-$C_{15}$ alkyle linéaire, ramifié ou cyclique en éventuellement substitué par des halogènes, groupes alcényle ou alcynyle, groupes alcaryle, groupes hétéroalkyle, groupes hétéroaryle, groupes hydroxy, disulfures, sulfonates, groupes éther, groupes thioéther, groupes ester, groupes acide carboxylique, groupes amine, groupes amide, azides ou benzoxazine, et $R_2$ peut être lié à $R_3$ pour former un substituant cyclique sur l'anneau benzénique ou $R_3$ peut être lié à $R_4$ pour former un substituant cyclique sur l'anneau benzénique, à condition que $R_2$, $R_3$, et/ou $R_4$ comprenne au moins une liaison insaturée ;
$R_7$ est $C_2$-$C_{15}$ un alkyle linéaire, ramifié ou cyclique en éventuellement substitué par des halogènes, des groupes alcaryle, des groupes hétéroalkyles, des groupes hétéroaryles, des groupes hydroxyles, des disulfures, des sulfonates, des groupes éthers, des groupes thioéthers, des groupes esters, des groupes acides carboxyliques, des groupes amines, des groupes amides et des azides, à condition que $R_7$ comprenne au moins un groupe thiol ;
$R_5$ et $R_6$ sont chacun indépendamment H, $CH_3$, $C_2$-$C_{15}$ alkyle linéaire, ramifié ou cyclique éventuellement substitué par des halogènes, alcényle, groupes hétéroalkyles et groupes hydroxy ; m est un nombre entier compris entre 10 et 10 000 ;
n est un nombre entier compris entre 10 et 6 700 ;
o est un nombre entier compris entre 0 et 1000.

**13.** Produit d'étanchéité, adhésif, revêtement, agent de liaison ou obturation dentaire comprenant une composition polymérisable expansible selon l'une quelconque des revendications 1 à 10.

**14.** Utilisation d'une composition polymérisable et expansible selon l'une quelconque des revendications 1 à 10 dans

ou en tant qu'agent d'étanchéité, adhésif, revêtement, agent de liaison ou obturation dentaire.

**15.** Utilisation d'une composition polymérisable et expansible comprenant au moins une benzoxazine et au moins un carbonate cyclique dans la fabrication d'un poly(benzoxazine)-co-poly(carbonate cyclique), dans laquelle la composition polymérisable et expansible présente une expansion volumétrique pendant la copolymérisation sous l'effet d'un stimulus thermique.

**16.** Utilisation d'une composition polymérisable et expansible selon la revendication 15, **caractérisée en ce que** la benzoxazine est un composé de la formule générale (**I**) :

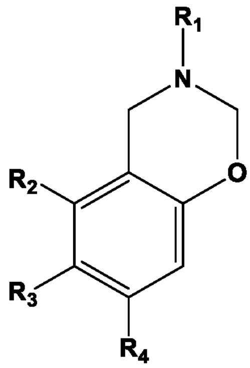

Formule (I)

dans lequel $R_1$, $R_2$, $R_3$, et $R_4$ sont chacun indépendamment H, $CH_3$, $C_2$-$C_{15}$ alkyle linéaire, ramifié ou cyclique éventuellement substitué par des halogènes, groupes alcényle ou alcynyle, groupes alcaryle, groupes hétéroalkyle, groupes hétéroaryle, groupes hydroxy, disulfures, sulfonates, groupes éther, groupes thioéther, groupes ester, groupes acide carboxylique, groupes amine, groupes amide, azides ou benzoxazines, et $R_2$ peut être lié à $R_3$ pour former un substituant cyclique sur l'anneau benzénique ou $R_3$ peut être lié à $R_4$ pour former un substituant cyclique sur l'anneau benzénique.

**17.** Utilisation d'une composition polymérisable et expansible selon la revendication 15 ou 16, **caractérisée en ce que** le carbonate cyclique est un composé de la formule générale (III) :

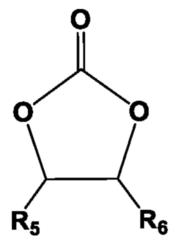

Formule (III)

dans lequel $R_5$ et $R_6$ sont chacun indépendamment H, $CH_3$, $C_2$-$C_{15}$ alkyle linéaire, ramifié ou cyclique éventuellement substitué par des halogènes, des groupes alcényles, hétéroalkyles et des groupes hydroxy.

**18.** Utilisation d'une composition expansible et polymérisable selon la revendication 17, **caractérisée en ce que** le carbonate cyclique est le carbonate d'éthylène ou le carbonate de propylène.

**19.** Utilisation d'une composition expansible et polymérisable selon l'une quelconque des revendications 15 à 18, **caractérisée en ce que** la benzoxazine est dérivée d'un dihydroxybenzène ou d'un bisphénol, de préférence choisi dans le groupe constitué par l'hydroquinone, le Bisphénol A, le Bisphénol F, le Bisphénol S, le Bisphénol M, le Bisphénol Z, le Bisphénol AP.

**20.** Utilisation d'une composition polymérisable et expansible selon l'une quelconque des revendications 15 à 19, **caractérisée en ce que** le rapport entre les équivalents de benzoxazine et les équivalents de carbonate cyclique dans la composition est compris entre 99:1 et 1:99, de préférence entre 99:1 et 30:70.

**21.** Utilisation d'une composition polymérisable expansible selon l'une quelconque des revendications 15 à 20, **caractérisée en ce que** la composition comprend en outre au moins un diluant réactif.

**22.** Utilisation d'une composition expansible et polymérisable selon la revendication 21, **caractérisée en ce que** le diluant réactif est présent à hauteur de 20 % à 60 % en poids de la benzoxazine.

**23.** Utilisation d'une composition expansible et polymérisable selon la revendication 21 ou 22, **caractérisée en ce que** le diluant réactif est choisi dans le groupe constitué de 3-allyl-3,4-dihydro-2H-benzo[e][1,3]oxazine, 3-allyl-5-methyl-3,4-dihydro-2H-benzo[e][1,3]oxazine, 3-allyl-6-octyl-3,4-dihydro-2H-benzo[e][1,3]oxazine and 3-allyl-6-nonyl-3,4-dihydro-2H-benzo[e][1,3]oxazine.

**24.** Utilisation d'une composition polymérisable et expansible selon l'une quelconque des revendications 15 à 23 dans ou en tant que les mastics, les adhésifs, les revêtements, les agents de liaison ou les obturations dentaires.

**25.** Produit de polymérisation poly(benzoxazine)-co-poly(carbonate cyclique) obtenu par copolymérisation d'une composition expansible et polymérisable telle que définie dans l'une quelconque des revendications 15 à 23 à une température suffisante pour initier la copolymérisation.

**26.** Produit de polymérisation poly(benzoxazine)-co-poly(carbonate cyclique) selon la revendication 25, comprenant un poly(benzoxazine)-co-poly(carbonate cyclique) de formule générale (**IV**) :

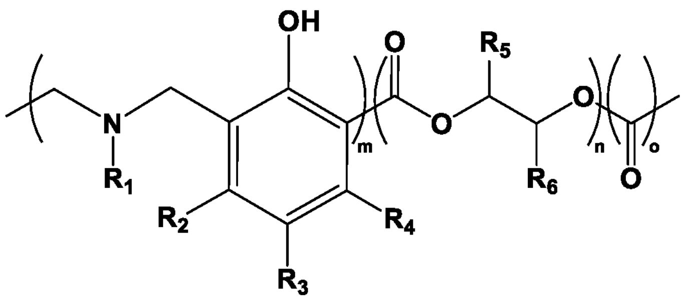

**Formule (IV)**

dans lequel

$R_1$, $R_2$, $R_3$, et $R_4$ sont chacun indépendamment H, $CH_3$, $C_2$-$C_{15}$ alkyle linéaire, ramifié ou cyclique éventuellement substitué par des halogènes, groupes alcényle ou alcynyle, groupes alcaryle, groupes hétéroalkyle, groupes hétéroaryle, groupes hydroxy, disulfures, sulfonates, groupes éther, groupes thioéther, groupes ester, groupes acide carboxylique, groupes amine, groupes amide, azides ou benzoxazine, et $R_2$ peut être lié à $R_3$ pour former un substituant cyclique sur l'anneau benzénique ou $R_3$ peut être lié à $R_4$ pour former un substituant cyclique sur l'anneau benzénique ;
$R_5$ et $R_6$ sont chacun indépendamment H, $CH_3$, $C_2$-$C_{15}$ alkyle linéaire, ramifié ou cyclique éventuellement substitué par des halogènes, des groupes alcényles, hétéroalkyles et des groupes hydroxy ;
m est un nombre entier compris entre 10 et 10 000 ;
n est un nombre entier compris entre 10 et 6700 ;
o est un nombre entier compris entre 0 et 1000.

**27.** Procédé de fabrication d'un produit de polymérisation poly(benzoxazine)-co-poly(carbonate cyclique), comprenant l'étape de chauffage d'une composition polymérisable expansible selon l'une quelconque des revendications 1 à 10 ou d'une composition polymérisable expansible telle que définie dans l'une quelconque des revendications 15 à 23 à une température suffisante pour initier la copolymérisation.

**28.** Procédé selon la revendication 27, comprenant les étapes suivantes :

- fournir une composition polymérisable selon l'une quelconque des revendications 1 à 10, comprenant des benzoxazines réticulables et des carbonates cycliques,
- préparer la composition polymérisable en réticulant les benzoxazines réticulables, et
- chauffer la composition polymérisable précuite à une température suffisante pour amorcer la copolymérisation des benzoxazines réticulées et des carbonates cycliques.

**29.** Composition polymérisable précuite et expansible obtenue par réticulation des benzoxazines réticulables contenues dans une composition polymérisable expansible selon l'une quelconque des revendications 1 à 10.

**30.** Utilisation d'une composition polymérisable précuite et expansible selon la revendication 29, comme élément de remplissage expansible pour remplir des espaces dans des dispositifs, dans laquelle la composition expansible précuite a une forme prédéfinie, et dans laquelle la composition expansible précuite présente une expansion volumétrique pendant la copolymérisation sous l'effet d'un stimulus thermique.

**31.** Utilisation d'une composition polymérisable précuite et expansible selon la revendication 30 comme élément de support et de fixation pour les enroulements des machines électriques ou comme barrière d'isolation des enroulements pour l'équipement électrique.

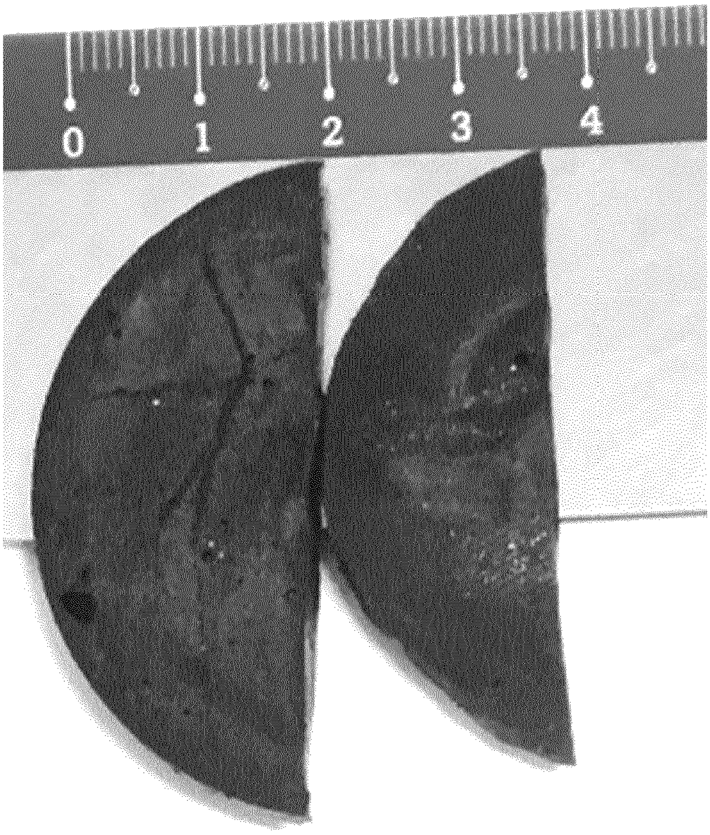


FIG. 1

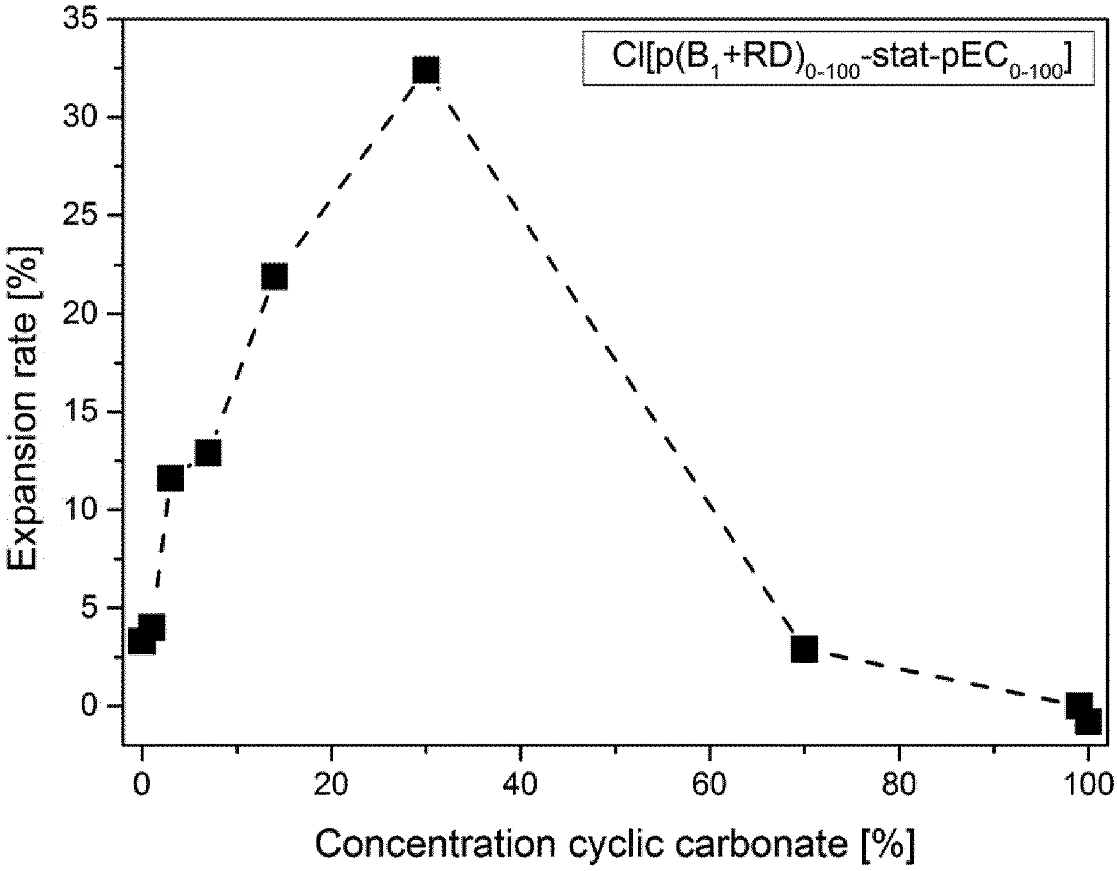
Cl[p(B1+RD)0-100-stat-pEC0-100]
Expansion rate [%]
Concentration cyclic carbonate [%]
35
30
25
20
15
10
5
0
0
20
40
60
80
100

FIG. 2

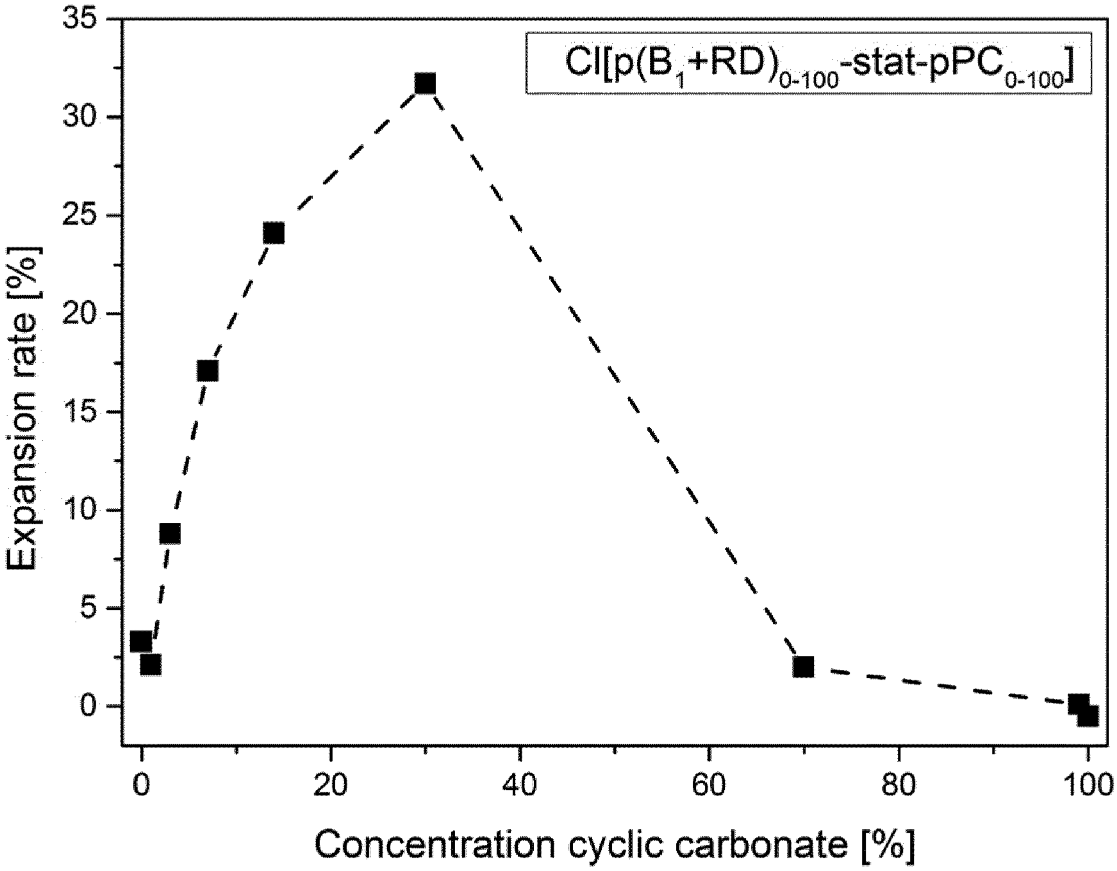
Cl[p(B1+RD)0-100-stat-pPC0-100]
Expansion rate [%]
Concentration cyclic carbonate [%]
35
30
25
20
15
10
5
0
0
20
40
60
80
100

FIG. 3

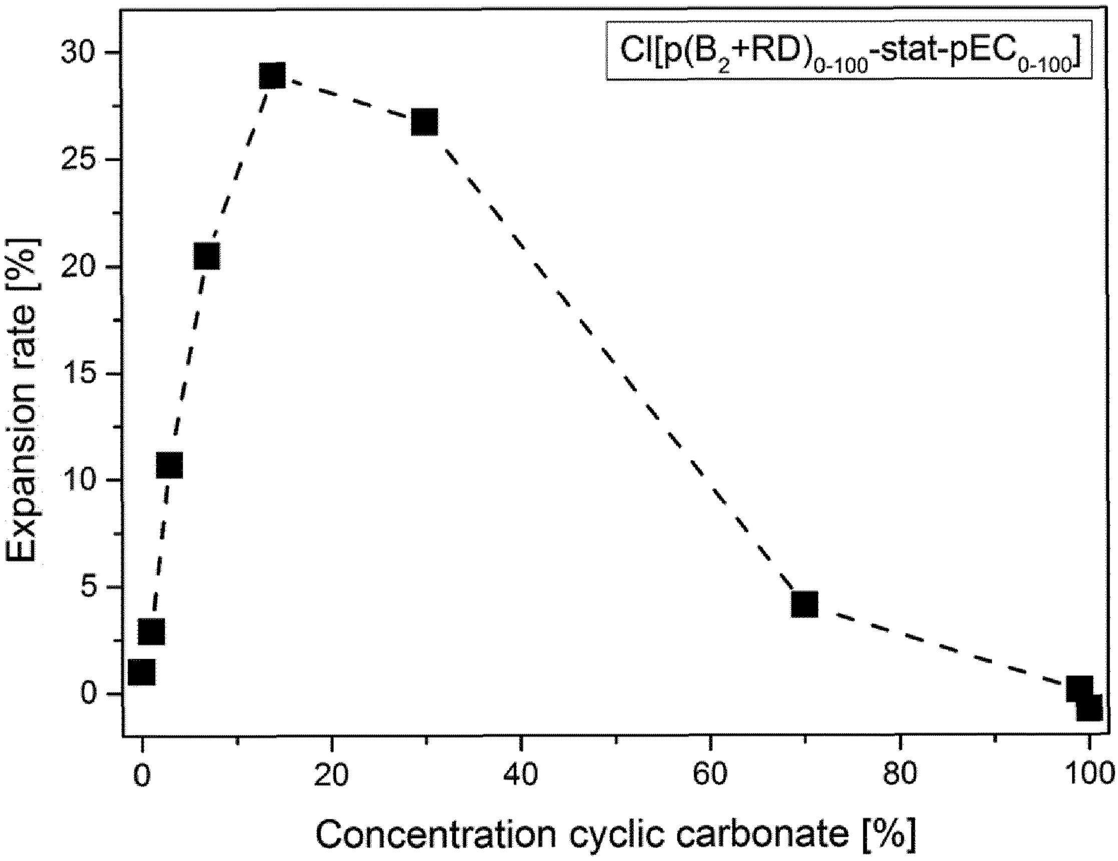
Cl[p(B2+RD)0-100-stat-pEC0-100]
Expansion rate [%]
30
25
20
15
10
5
0
0
20
40
60
80
100
Concentration cyclic carbonate [%]

FIG. 4

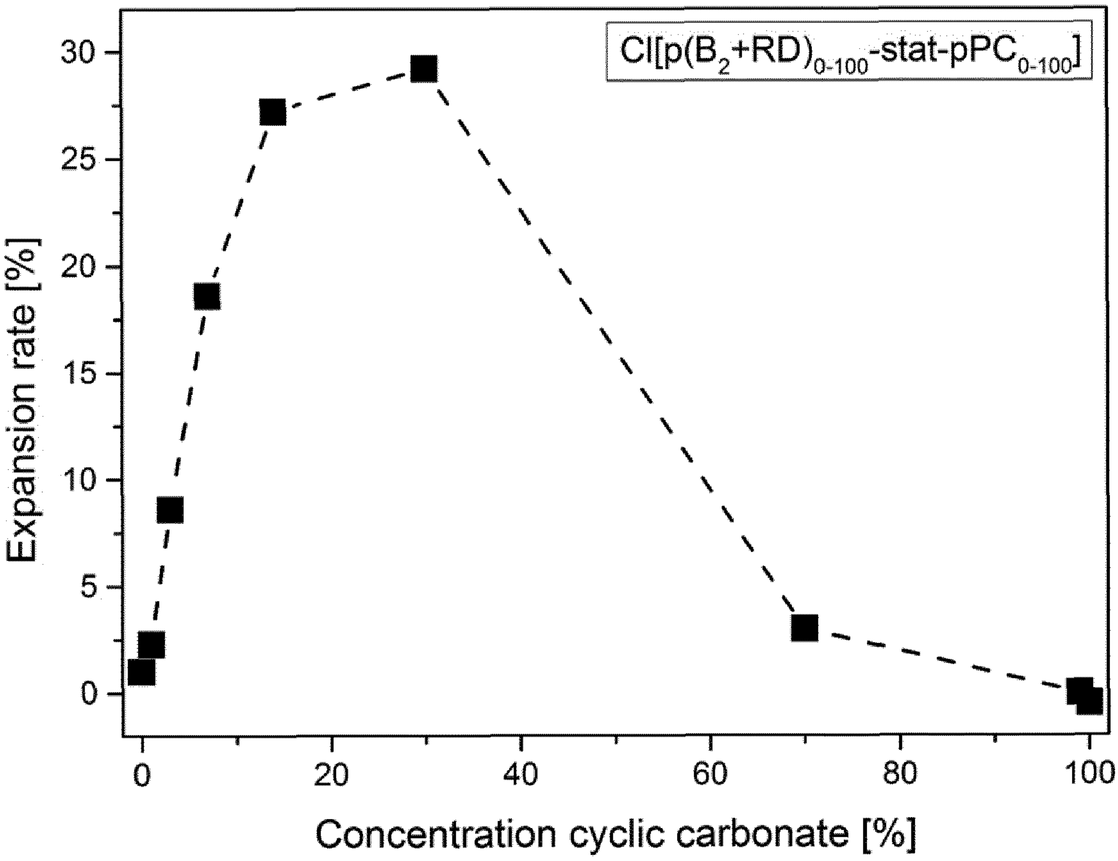
Cl[p(B2+RD)0-100-stat-pPC0-100]
Expansion rate [%]
Concentration cyclic carbonate [%]
0
5
10
15
20
25
30
0
20
40
60
80
100

FIG. 5

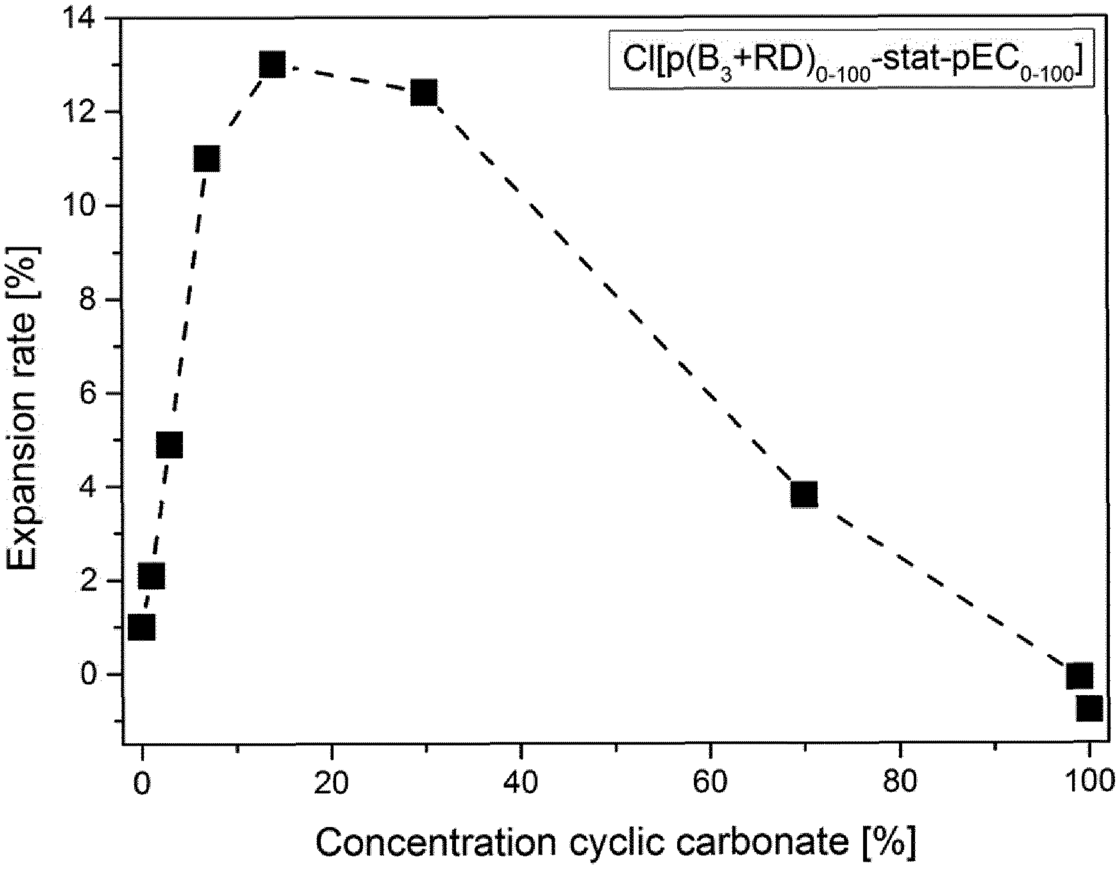
Cl[p(B3+RD)0-100-stat-pEC0-100]
Expansion rate [%]
14
12
10
8
6
4
2
0
0
20
40
60
80
100
Concentration cyclic carbonate [%]

FIG. 6

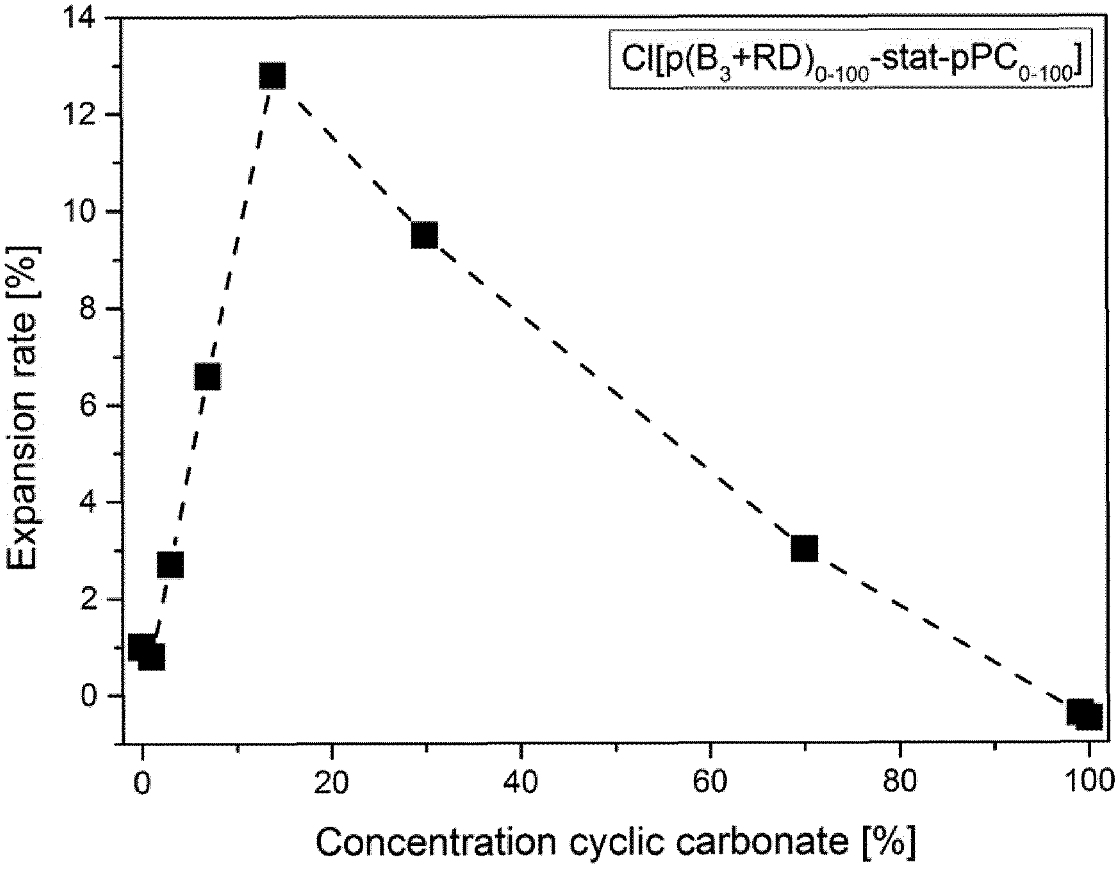
Cl[p(B3+RD)0-100-stat-pPC0-100]
Expansion rate [%]
14
12
10
8
6
4
2
0
0
20
40
60
80
100
Concentration cyclic carbonate [%]

FIG. 7

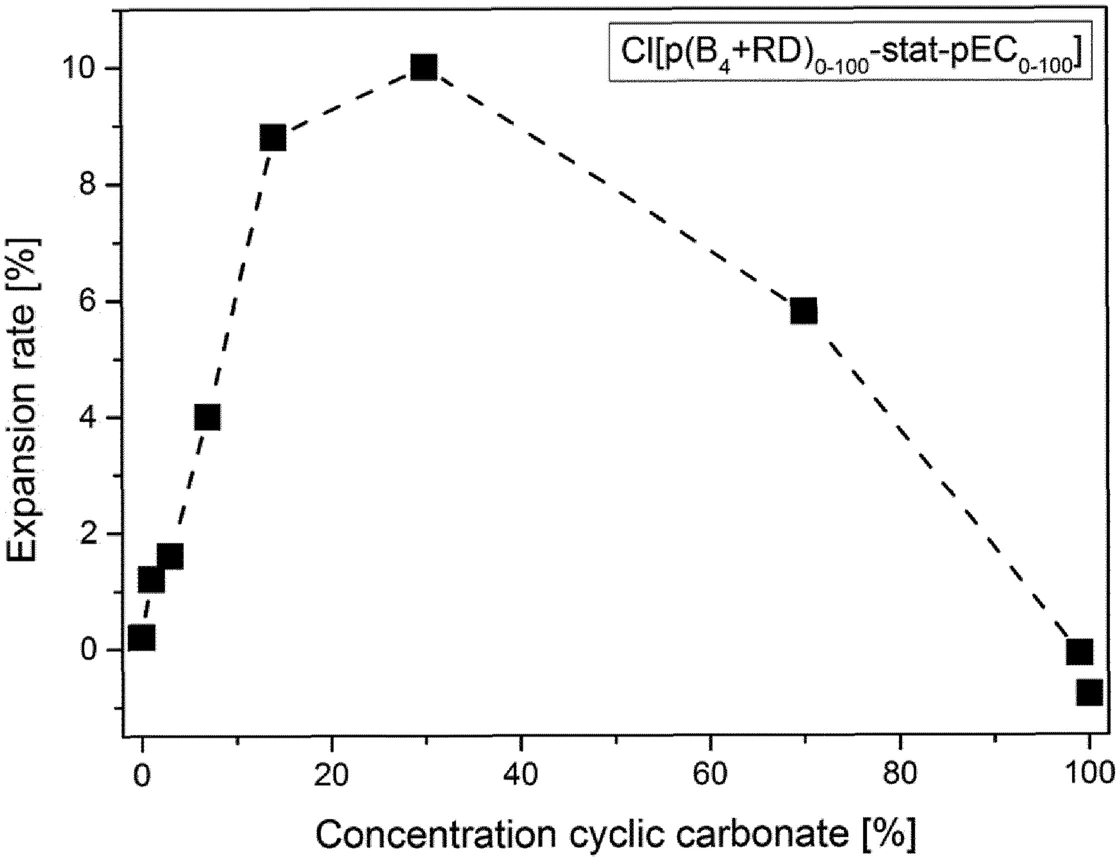
Cl[p(B4+RD)0-100-stat-pEC0-100]
Expansion rate [%]
10
8
6
4
2
0
0
20
40
60
80
100
Concentration cyclic carbonate [%]

FIG. 8

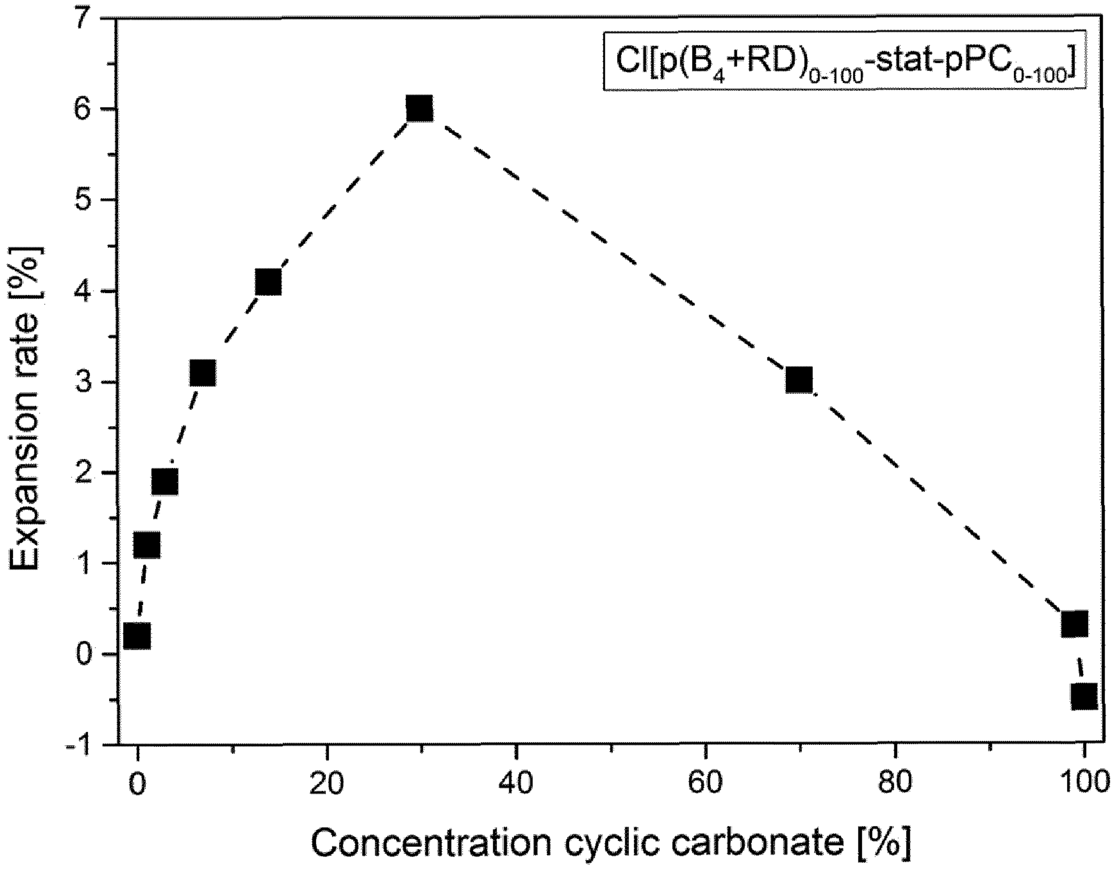
Cl[p(B4+RD)0-100-stat-pPC0-100]
Expansion rate [%]
Concentration cyclic carbonate [%]
7
6
5
4
3
2
1
0
-1
0
20
40
60
80
100

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2560227 A1
- WO 2018229415 A1


### Non-patent literature cited in the description


- **T. TAKATA ; T. ENDO.** Recent advances in the development of expanding monomers: Synthesis, polymerization and volume change. Prog. *Polym. Sci.,* 1993, vol. 18 (5), 839-870
- **H. ISHIDA ; H. Y. LOW.** A Study on the Volumetric Expansion of Benzoxazine-Based Phenolic Resin. *Macromolecules,* 1997, vol. 30, 1099-1106
- **M. MURAYAMA.** Anionic Ring-Opening Polymerization of a Cyclic Carbonate Having a Norbornene Structure with Amine Initiators. *Macromolecules,* 1998, vol. 31, 919-923
- **L.W. CHEN ; C.H. TWU ; C.S. CHO.** Physical properties and shrinkage of epoxy resins cured with lactams. *J. Polym. Res.,* 1997, vol. 4, 65-72
- **L. QIN ; J. NIE ; Y. HE.** Synthesis and properties of polyurethane acrylate modified by different contents of stearyl alcohol. *J. Coat. Technol. Res.,* 2015, vol. 12 (19), 7-204
- **J. GE ; M. TRUJILLO ; J. STANSBURY.** Synthesis and photopolymerization of low shrinkage methacrylate monomers containing bulky substituent groups. *Dent. Mater.,* 2005, vol. 21, 1163-1169
- **B. LU ; P. XIAO ; M. SUN.** J. Nie: Reducing volume shrinkage by low-temperature photopolymerization. *J. Appl. Polym. Sci.,* 2007, vol. 104, 1126-1130
- **Y. JIAN ; Y. HE ; T. JIANG ; C. LI, W. YANG ; J. NIE.** Volume shrinkage of UV-curable coating formulation investigated by real-time laser reflection method. *J. Coat. Technol. Res.,* 2013, vol. 10, 231-237
- **T. LI ; H. QIN ; Y. LIU ; Y. ZHONG ; Y. YU ; A. SERRA.** Hyperbranched polyester as additives in filled and unfilled epoxy-novolac systems. *Polymer,* 2012, vol. 53 (25), 5864-5872
- **J. PARAMESWARANPILLAI ; A. GEORGE ; J. PIONTECK ; S. THOMAS.** Investigation of Cure Reaction, Rheology, Volume Shrinkage and Thermomechanical Properties of Nano-TiO2 Filled Epoxy/DDS Composites. *J. Polym.,* 2013, vol. 183463, 183463-1, 18363-17